# EUROPEAN PATENT APPLICATION

(11) **EP 1 854 878 A1**
(43) Date of publication of application: **14.11.2007**
(21) Application number: 06113772.5
(22) Date of filing: 10.05.2006
(51) Int. Cl.: C12N 9/88, C12N 15/81, C12N 15/60, C12R 1/84

(54) **Methods and materials for the transformation of the yeast Pichia ciferrii**

(71) Applicant: Cosmoferm B.V., 2600 AJ Delft (NL)
(72) Inventor: Boergel, Daniel, 1190 Wien (AT); Hueller, Thomas, 63454 Hanau (DE); Schaffer, Steffen, 63450 Hanau (DE)
(74) Representative: Aalbers, Arnt Reinier

(57) **Abstract**

The present invention discloses a transformation system useful for the genetic engineering of the industrial yeast *Pichia ciferrii.* It describes the isolation of auxotrophic mutants of this yeast and nucleotide sequences able to complement the auxotrophic requirements of these strains. The transformation system is based on mutant *Pichia ciferrii* cells auxotrophic for uracil or lysine, which are transformed with plasmids containing the *URA3* or *LYS2* genes as selection markers. Novel *URA3* and *LYS2* genes are also disclosed.

SEQUENCE LISTING

<110>  Cosmoferm B.V.

<120>  Methods and materials for the transformation of the yeast Pichia
       ciferrii

<130>  45

<160>  4

<170>  PatentIn version 3.3

<210>  1
<211>  2359
<212>  DNA
<213>  Pichia ciferrii


<220>
<221>  CDS
<222>  (1072)..(1875)
<223>  Product: Orotidine-5'-phosphate decarboxylase

<400>  1
aacaatttaa tactgaaata aaagttgtaa tatagtataa aatataaaat tcatgagaaa      60

tatgtacaaa aggtttgtaa gatcaccaca ttttctttaa aatgatctca ccataataat     120

aaaacatcta aagataaatc aaagggaacc cacaaattga aatgaaaaga attgtataaa     180

catcatgact ttaaacaatt cacttctagt atatgagaga taataaattc attaacagta     240

tttttttttt ctaaattttt taattttttk ywtstamrkk mywyrakymw ayrarwawaw     300

wwgwyyratr mryykrwtaa atatctgaac cttaacgtct catttgttga gttaataaag     360

cattttgttc ttggaatctt tgaccgactc tttcactgtg tttcaagaat ttttcagaac     420

attttaaaac acatgtattt tcttttgaag ttaatgaagc ggaagtgaaa tcattaacac     480

aatcattgaa acaacgttca accaagtttg agtaaagacg catgaaatct ttcatttgtt     540

taccttcaac gattctttgg aaatcttgtt gttctctttg gtttaattga tccattttga     600

ttgatttgat atagatttga gctttgtagt tctgatcaat taggtacaaa aggtctttca     660

atggcaaaag tgtacttttt tgatttttca atgaagtcga aaaggttcaa attttttttt     720

ggtcaacaaa acatcataaa tatccgaatt tggaaagatt cccgatatgg acaaaatcag     780

gaatcaattg gaattatgat tcatttgagc attatagtgt gaataattct ttatgttctc     840

tgttctttat tatatcaagc tttaaagaga cactaggcgt cccatagatc ggatggtcta     900

ttcaagtgtc tcaaagaaaa aaaatagttg cttgattttg gctgtagtag agcttgtacg     960

ggacacgctt ctatgaataa atttgtccga tgagttggat aaatattaac tgaactaatt    1020

tcttctaatt gatagagcat catcaatata ttgcacataa tataattcaa a atg gtt    1077
                                                         Met Val

acc aca tta tct tat tct caa aga gca gaa gct cac cct tct cca ttg 1125
Thr Thr Leu Ser Tyr Ser Gln Arg Ala Glu Ala His Pro Ser Pro Leu
        5                   10                  15

gca aag cgt tta ttc aat gtt atg gaa tca aaa aag tca aac ttg tgt 1173
Ala Lys Arg Leu Phe Asn Val Met Glu Ser Lys Lys Ser Asn Leu Cys
    20                  25                  30

gct tca att gat gtt aga act aca aaa gaa ttt tta caa tta gtt gac 1221
Ala Ser Ile Asp Val Arg Thr Thr Lys Glu Phe Leu Gln Leu Val Asp
35                  40                  45                  50

aca tta ggt cca tat atc tgt ctt gtc aaa act cat atc gat att att 1269
Thr Leu Gly Pro Tyr Ile Cys Leu Val Lys Thr His Ile Asp Ile Ile
                55                  60                  65

gat gat ttc tca ttc gaa ggt acc att aaa cca ttg aaa gaa tta gca 1317
Asp Asp Phe Ser Phe Glu Gly Thr Ile Lys Pro Leu Lys Glu Leu Ala
            70                  75                  80

caa aag cac aat ttt tta atc ttt gaa gat cgt aaa ttt gct gat ata 1365
Gln Lys His Asn Phe Leu Ile Phe Glu Asp Arg Lys Phe Ala Asp Ile
        85                  90                  95

ggg aac aca gtc aaa gca caa tat gca ggt ggt gct ttc aaa att gca 1413
Gly Asn Thr Val Lys Ala Gln Tyr Ala Gly Gly Ala Phe Lys Ile Ala
    100                 105                 110

act tgg tct gat atc aca aat gct cat ggt gtt act ggt gct ggt atc 1461
Thr Trp Ser Asp Ile Thr Asn Ala His Gly Val Thr Gly Ala Gly Ile
115                 120                 125                 130

gtt agt gga ttg aaa gaa gct gct aaa gaa gct tct gat gaa cca aga 1509
Val Ser Gly Leu Lys Glu Ala Ala Lys Glu Ala Ser Asp Glu Pro Arg
                135                 140                 145

ggg ttg ttg atg ttg gct gaa ttg agt tca aaa ggt tca tta gcc aaa 1557
Gly Leu Leu Met Leu Ala Glu Leu Ser Ser Lys Gly Ser Leu Ala Lys
            150                 155                 160

ggt gaa tat act aaa caa act gtt gat atc gct aaa act gat aaa gat 1605
Gly Glu Tyr Thr Lys Gln Thr Val Asp Ile Ala Lys Thr Asp Lys Asp
        165                 170                 175

ttc gtt att ggt ttt att gct caa aat gat atg ggt ggt aga gat gga 1653
Phe Val Ile Gly Phe Ile Ala Gln Asn Asp Met Gly Gly Arg Asp Gly
    180                 185                 190

ggc ttt gat tgg tta att atg acc cca ggt gtt ggt ctt gat gat aaa 1701
Gly Phe Asp Trp Leu Ile Met Thr Pro Gly Val Gly Leu Asp Asp Lys
195                 200                 205                 210

ggt gat gcc tta ggt caa caa tat aga tcc gtt gat acc gtt gtt tca 1749
Gly Asp Ala Leu Gly Gln Gln Tyr Arg Ser Val Asp Thr Val Val Ser
                215                 220                 225

act ggt agt gat atc att att gtt ggt aga ggt ttg ttc ggt aaa gga 1797
Thr Gly Ser Asp Ile Ile Ile Val Gly Arg Gly Leu Phe Gly Lys Gly
            230                 235                 240

aga gat cca aat gct gaa ggt gaa cgt tac aga gct gct ggt tgg aat     1845
Arg Asp Pro Asn Ala Glu Gly Glu Arg Tyr Arg Ala Ala Gly Trp Asn
        245                 250                 255

gcc tat ttg aaa aga act ggt caa ata tag tatgtagttt aaatctaaca     1895
Ala Tyr Leu Lys Arg Thr Gly Gln Ile
    260                 265

tattagacta aataattaat gggtcataat ttgtaattag ttcaacgact aactaactga     1955

cattagaagg aagactctaa ttgtatacca tgtcatcaat actcctaatt atcaaataaa     2015

tcaaaatata taccttctat cattaattag aagatgtaat gtcgatgact aagcaagata     2075

aattaaaacc gaaatccatc tgtatgtaaa aatactttca agtaattacc acatagatag     2135

aagctcacca accaactatc atcaagccag tagatgccat tcaagagtga caaatgtcat     2195

tggaaaatga gttcacagga tcctcctttg agaatgagtt tgaaaatact actgcccaac     2255

cagaattatc aaataaagga tcgagattga ttggtgctag taccgcaggt ataagagctc     2315

taatgtatca attaaccact atatacttaa gaacccctcc agca     2359

<210>  2
<211>  267
<212>  PRT
<213>  Pichia ciferrii

<400>  2

Met Val Thr Thr Leu Ser Tyr Ser Gln Arg Ala Glu Ala His Pro Ser
1               5                   10                  15

Pro Leu Ala Lys Arg Leu Phe Asn Val Met Glu Ser Lys Lys Ser Asn
            20                  25                  30

Leu Cys Ala Ser Ile Asp Val Arg Thr Thr Lys Glu Phe Leu Gln Leu
        35                  40                  45

Val Asp Thr Leu Gly Pro Tyr Ile Cys Leu Val Lys Thr His Ile Asp
    50                  55                  60

Ile Ile Asp Asp Phe Ser Phe Glu Gly Thr Ile Lys Pro Leu Lys Glu
65                  70                  75                  80

Leu Ala Gln Lys His Asn Phe Leu Ile Phe Glu Asp Arg Lys Phe Ala
                85                  90                  95

Asp Ile Gly Asn Thr Val Lys Ala Gln Tyr Ala Gly Gly Ala Phe Lys
            100                 105                 110

Ile Ala Thr Trp Ser Asp Ile Thr Asn Ala His Gly Val Thr Gly Ala
        115                 120                 125

Gly Ile Val Ser Gly Leu Lys Glu Ala Ala Lys Glu Ala Ser Asp Glu
    130                 135                 140

Pro Arg Gly Leu Leu Met Leu Ala Glu Leu Ser Ser Lys Gly Ser Leu
145                 150                 155                 160

Ala Lys Gly Glu Tyr Thr Lys Gln Thr Val Asp Ile Ala Lys Thr Asp
                165                 170                 175

Lys Asp Phe Val Ile Gly Phe Ile Ala Gln Asn Asp Met Gly Gly Arg
            180                 185                 190

Asp Gly Gly Phe Asp Trp Leu Ile Met Thr Pro Gly Val Gly Leu Asp
        195                 200                 205

Asp Lys Gly Asp Ala Leu Gly Gln Gln Tyr Arg Ser Val Asp Thr Val
    210                 215                 220

Val Ser Thr Gly Ser Asp Ile Ile Ile Val Gly Arg Gly Leu Phe Gly
225                 230                 235                 240

Lys Gly Arg Asp Pro Asn Ala Glu Gly Glu Arg Tyr Arg Ala Ala Gly
                245                 250                 255

Trp Asn Ala Tyr Leu Lys Arg Thr Gly Gln Ile
            260                 265

<210>  3
<211>  5900
<212>  DNA
<213>  Pichia ciferrii

<220>
<221>  CDS
<222>  (810)..(4997)
<223>  Product: alpha-aminoadipate reductase

<400>  3
aattattctt atctttaaca tcattaccac ttaaccaatt ctttatttga tctctttgtc      60

tttgtaattt cttgatttct cttttcaaat caccttcaat tttttccttt tgagactgac     120

tttcacaatt ttgaagcttg tcatagtagc catcaaagat ctctagacct tctttaactt     180

tcttgaagac tttatcaatc tcttgtaata attttctttg tgacattgtt aaatctttgt     240

ttaaatctgt atatgatatg aaactgtgtg tttatcgaag cacttttctc actcaattgg     300

aacgttttgg acttgtaaga aaaccagcgg atagactatt gaacacgtag aattcaaaaa 360

caattaaaaa acagcacaat taccaactat tcaacacaga attcaccgtt gtttgatcca 420

ccaaaattgt cacgtctcta ttattccagc gcttttttct gattctttcc actttttttg 480

gtgagcaaat cgagatccga ccatttttaa agatcggaac tcccggtttt ctagagtttt 540

ttttttgaca gccgatttcc gaccgatttt ttgagattat cagtcataat gtttttgatg 600

aaagattttg attgtatttg atattgttaa ctaagtggta agagttcaag caaggtttta 660

tcttggttta aagtgagtaa aagtggtatt gtatcaggaa ttatcaccat tgtgctttgt 720

ttcgaggatc attggtgtaa atttcatcca tttcacaagg atctcttgta ctaacatttc 780

caattgatat agatacattt tattcaaaa atg tct tca gat tta caa cat tgg 833
Met Ser Ser Asp Leu Gln His Trp
1               5

gct caa ata ttg agt agt cca act ctc tca gta tta cct cat gat ttc 881
Ala Gln Ile Leu Ser Ser Pro Thr Leu Ser Val Leu Pro His Asp Phe
    10                  15                  20

att cgt cct tct aac aac aaa gtt gtt gaa gct aaa cac aat ttt tca 929
Ile Arg Pro Ser Asn Asn Lys Val Val Glu Ala Lys His Asn Phe Ser
25                  30                  35                  40

att cca gca aaa gca tta tca caa tct ata ata tcg aaa gtt gaa ggt 977
Ile Pro Ala Lys Ala Leu Ser Gln Ser Ile Ile Ser Lys Val Glu Gly
                45                  50                  55

tca aat gaa ttc atc att ggt ctt tca gtc ttt gca gtt ttg atc tat 1025
Ser Asn Glu Phe Ile Ile Gly Leu Ser Val Phe Ala Val Leu Ile Tyr
            60                  65                  70

aga cta act ggt gat gat gac att gtt atc tcc acc gac tct cca aat 1073
Arg Leu Thr Gly Asp Asp Asp Ile Val Ile Ser Thr Asp Ser Pro Asn
        75                  80                  85

ggc ggt aaa gaa ttt gtc att aga tta acc att agt cca act gtt tca 1121
Gly Gly Lys Glu Phe Val Ile Arg Leu Thr Ile Ser Pro Thr Val Ser
    90                  95                  100

ttt gtg gaa tta gtt gaa caa att aca aaa tta tat aat gac aat gtc 1169
Phe Val Glu Leu Val Glu Gln Ile Thr Lys Leu Tyr Asn Asp Asn Val
105                 110                 115                 120

aat aga gct gtt gat tat gat gaa tta tct gca ttg atc caa aaa gaa 1217
Asn Arg Ala Val Asp Tyr Asp Glu Leu Ser Ala Leu Ile Gln Lys Glu
                125                 130                 135

aac ggt tat gaa act aaa cct tct tta ttc aga tta agt ttc caa tat 1265
Asn Gly Tyr Glu Thr Lys Pro Ser Leu Phe Arg Leu Ser Phe Gln Tyr
            140                 145                 150

ggt aat gct cat caa caa tta tct gaa tat ggt tca att aga gat tta 1313
Gly Asn Ala His Gln Gln Leu Ser Glu Tyr Gly Ser Ile Arg Asp Leu
        155                 160                 165

tca atc ttt gtc aat gat gat tct tat gat gtc tat ttt aat gct tta 1361
Ser Ile Phe Val Asn Asp Asp Ser Tyr Asp Val Tyr Phe Asn Ala Leu
    170                 175                 180

tta tac aaa gat gat cgt att tca att ttg gct gaa caa ttt aac caa 1409
Leu Tyr Lys Asp Asp Arg Ile Ser Ile Leu Ala Glu Gln Phe Asn Gln
185                 190                 195                 200

ttt ttg gaa gct gtc gcc caa gat tct tca tta caa gtt act aaa ata 1457
Phe Leu Glu Ala Val Ala Gln Asp Ser Ser Leu Gln Val Thr Lys Ile
                205                 210                 215

aat tta atc acc aaa tct caa aaa caa atc tta cca gat cca aca act 1505
Asn Leu Ile Thr Lys Ser Gln Lys Gln Ile Leu Pro Asp Pro Thr Thr
            220                 225                 230

gat tta gat tgg tct ggt tat aga ggt gct atc cat gat att ttc caa 1553
Asp Leu Asp Trp Ser Gly Tyr Arg Gly Ala Ile His Asp Ile Phe Gln
        235                 240                 245

gaa aat gca gct aaa ttc cca gaa cgt act tgt gtt gtg gaa act cca 1601
Glu Asn Ala Ala Lys Phe Pro Glu Arg Thr Cys Val Val Glu Thr Pro
    250                 255                 260

agt ttc tta aca cca gaa aaa caa gct aga gtc ttt act tat aaa cat 1649
Ser Phe Leu Thr Pro Glu Lys Gln Ala Arg Val Phe Thr Tyr Lys His
265                 270                 275                 280

atc aat gaa gct tca aac att gtt ggt aac tat tta gtt aaa act ggt 1697
Ile Asn Glu Ala Ser Asn Ile Val Gly Asn Tyr Leu Val Lys Thr Gly
                285                 290                 295

atc aaa aga ggt gat gtt gtt atg att tat tct tca aga ggt gtt gat 1745
Ile Lys Arg Gly Asp Val Val Met Ile Tyr Ser Ser Arg Gly Val Asp
            300                 305                 310

tta atg gtt tca gtt ctc ggt gtt tta aaa gcc ggt gct act ttc tcc 1793
Leu Met Val Ser Val Leu Gly Val Leu Lys Ala Gly Ala Thr Phe Ser
        315                 320                 325

gtt att gat cca gct tat cct cca gct cgt caa aat att tat tta caa 1841
Val Ile Asp Pro Ala Tyr Pro Pro Ala Arg Gln Asn Ile Tyr Leu Gln
    330                 335                 340

gtt gct aaa cca aaa ggt tta atc gtc atc aaa aaa gct ggt gtc ttg 1889
Val Ala Lys Pro Lys Gly Leu Ile Val Ile Lys Lys Ala Gly Val Leu
345                 350                 355                 360

gat gaa ctt gtt gaa aat tac atc aaa gat gaa tta gat gtt gtt tct 1937
Asp Glu Leu Val Glu Asn Tyr Ile Lys Asp Glu Leu Asp Val Val Ser
                365                 370                 375

cgt gtt cct caa att gaa tta caa gat gat ggt tca gtc ctc ggt ggt 1985
Arg Val Pro Gln Ile Glu Leu Gln Asp Asp Gly Ser Val Leu Gly Gly
            380                 385                 390

aaa aac ttg gaa ggt gaa gat gtc tta gct ggt gtt tca aca agt tca 2033
Lys Asn Leu Glu Gly Glu Asp Val Leu Ala Gly Val Ser Thr Ser Ser
        395                 400                 405

act ggt gtt ttg gtt ggt cca gat tca aac cca aca tta tct ttc aca 2081

Thr Gly Val Leu Val Gly Pro Asp Ser Asn Pro Thr Leu Ser Phe Thr
    410                 415                 420

tca ggt tct gaa ggt tta cca aaa ggt gtc ttg ggt aga cat ttc tct      2129
Ser Gly Ser Glu Gly Leu Pro Lys Gly Val Leu Gly Arg His Phe Ser
425                 430                 435                 440

tta gct tat tat ttc gat tgg atg tcc aag act ttt aaa tta aac tct      2177
Leu Ala Tyr Tyr Phe Asp Trp Met Ser Lys Thr Phe Lys Leu Asn Ser
                445                 450                 455

gat gat aaa ttc act atg ctt tca ggt att gca cat gat cct att caa      2225
Asp Asp Lys Phe Thr Met Leu Ser Gly Ile Ala His Asp Pro Ile Gln
            460                 465                 470

aga gat atg ttt aca cca atc ttc ttg ggt gct caa tta tta gtt cca      2273
Arg Asp Met Phe Thr Pro Ile Phe Leu Gly Ala Gln Leu Leu Val Pro
        475                 480                 485

act gct gat gat atc ggt acc cca ggt aaa tta gct gaa tgg atg ggt      2321
Thr Ala Asp Asp Ile Gly Thr Pro Gly Lys Leu Ala Glu Trp Met Gly
    490                 495                 500

aaa tac ggt gct act gtt act cat tta act cca gct atg ggt caa tta      2369
Lys Tyr Gly Ala Thr Val Thr His Leu Thr Pro Ala Met Gly Gln Leu
505                 510                 515                 520

tta tca gct caa gct aca tct gaa att cca tca tta cat cat gct ttc      2417
Leu Ser Ala Gln Ala Thr Ser Glu Ile Pro Ser Leu His His Ala Phe
                525                 530                 535

ttt gta ggt gat atc ttg acc aaa aga gat tgt tta aga tta caa aca      2465
Phe Val Gly Asp Ile Leu Thr Lys Arg Asp Cys Leu Arg Leu Gln Thr
            540                 545                 550

tta gct aaa aat gtt ttc att gtc aat atg tat ggt act act gaa act      2513
Leu Ala Lys Asn Val Phe Ile Val Asn Met Tyr Gly Thr Thr Glu Thr
        555                 560                 565

caa aga gct gtt tca tat ttt gaa gtt gct tca aga gct cat gat tca      2561
Gln Arg Ala Val Ser Tyr Phe Glu Val Ala Ser Arg Ala His Asp Ser
    570                 575                 580

aca ttt tta gaa tct caa aaa gat gtc atg cca gct ggt aaa ggt atg      2609
Thr Phe Leu Glu Ser Gln Lys Asp Val Met Pro Ala Gly Lys Gly Met
585                 590                 595                 600

ttg aat gtt caa tta tta gtt gtt aat aga aat gat act tct caa aca      2657
Leu Asn Val Gln Leu Leu Val Val Asn Arg Asn Asp Thr Ser Gln Thr
                605                 610                 615

tgt ggt gtt ggt gaa gtt ggt gaa att tat gtt cgt gct gct ggt tta      2705
Cys Gly Val Gly Glu Val Gly Glu Ile Tyr Val Arg Ala Ala Gly Leu
            620                 625                 630

gct gaa ggt tat cgt gga tta cct gaa tta aat gaa caa aaa ttc gtc      2753
Ala Glu Gly Tyr Arg Gly Leu Pro Glu Leu Asn Glu Gln Lys Phe Val
        635                 640                 645

aaa aat tgg tat gtt gat gct caa caa tgg att gat caa gat aat caa      2801
Lys Asn Trp Tyr Val Asp Ala Gln Gln Trp Ile Asp Gln Asp Asn Gln

650                     655                     660

aaa agt caa aat gaa cca tgg aga gaa ttt tgg aaa ggt cca aga gat     2849
Lys Ser Gln Asn Glu Pro Trp Arg Glu Phe Trp Lys Gly Pro Arg Asp
665                 670                 675                 680

aga tta tac aga act ggt gat tta ggt cgt tat tta cca gat ggt aat     2897
Arg Leu Tyr Arg Thr Gly Asp Leu Gly Arg Tyr Leu Pro Asp Gly Asn
                685                 690                 695

act gaa tgt tgt ggt aga gct gat gat caa gtc aaa att cgt ggg ttc     2945
Thr Glu Cys Cys Gly Arg Ala Asp Asp Gln Val Lys Ile Arg Gly Phe
            700                 705                 710

cgt att gaa tta ggt gaa att gat aca aat att tct caa cat cct tta     2993
Arg Ile Glu Leu Gly Glu Ile Asp Thr Asn Ile Ser Gln His Pro Leu
        715                 720                 725

gtt cgt gag aat gtt aca tta gtt cgt aaa gat gct aat aat gaa cca     3041
Val Arg Glu Asn Val Thr Leu Val Arg Lys Asp Ala Asn Asn Glu Pro
    730                 735                 740

act tta att tct tat att gtt cca aaa act gaa gtt gct gat tta gaa     3089
Thr Leu Ile Ser Tyr Ile Val Pro Lys Thr Glu Val Ala Asp Leu Glu
745                 750                 755                 760

aat ttc aaa tca tca gtt gaa gaa tta gaa aat aaa gat gat cac gtt     3137
Asn Phe Lys Ser Ser Val Glu Glu Leu Glu Asn Lys Asp Asp His Val
                765                 770                 775

gtt agt gga tta gtt ggt tat cgt tta tta att aaa gat atc aag gca     3185
Val Ser Gly Leu Val Gly Tyr Arg Leu Leu Ile Lys Asp Ile Lys Ala
            780                 785                 790

ttt ttg aaa aaa aga tta gct tca tat gct atc cca aca att att gtt     3233
Phe Leu Lys Lys Arg Leu Ala Ser Tyr Ala Ile Pro Thr Ile Ile Val
        795                 800                 805

cca ttc cat aaa tta cca tta aat cca aat ggt aaa gtt gat aaa cca     3281
Pro Phe His Lys Leu Pro Leu Asn Pro Asn Gly Lys Val Asp Lys Pro
    810                 815                 820

aaa tta cca ttc cca acc act gca caa cta gaa gct gtt gct aaa cat     3329
Lys Leu Pro Phe Pro Thr Thr Ala Gln Leu Glu Ala Val Ala Lys His
825                 830                 835                 840

tca agt gcc gac caa gat gat tct caa ttc agt gaa act gaa ggt aaa     3377
Ser Ser Ala Asp Gln Asp Asp Ser Gln Phe Ser Glu Thr Glu Gly Lys
                845                 850                 855

gtt cgt gat gtt tgg tta tca gtc tta cct caa cgt cca tca act att     3425
Val Arg Asp Val Trp Leu Ser Val Leu Pro Gln Arg Pro Ser Thr Ile
            860                 865                 870

tca cca gaa gat tca ttc ttt gat tta ggt ggt cat tca atc tta gct     3473
Ser Pro Glu Asp Ser Phe Phe Asp Leu Gly Gly His Ser Ile Leu Ala
        875                 880                 885

aca aga atg att ttt gaa tta aga aaa caa tta gtt att gat tta cca     3521
Thr Arg Met Ile Phe Glu Leu Arg Lys Gln Leu Val Ile Asp Leu Pro
    890                 895                 900

tta ggt act atc ttc aaa ttc cca acc att aaa gct ttt gct aaa gaa 3569
Leu Gly Thr Ile Phe Lys Phe Pro Thr Ile Lys Ala Phe Ala Lys Glu
905                 910                 915                 920

atc gaa cgt gtt caa aaa ggt gat gat gtc gcc tta cca gct act agt 3617
Ile Glu Arg Val Gln Lys Gly Asp Asp Val Ala Leu Pro Ala Thr Ser
                925                 930                 935

ggt gct gaa aat act gat gag gat tta gca aat aaa cca gct gaa tat 3665
Gly Ala Glu Asn Thr Asp Glu Asp Leu Ala Asn Lys Pro Ala Glu Tyr
            940                 945                 950

tat aaa gat gct caa gaa tta act gaa act gca tta aaa tca aaa tat 3713
Tyr Lys Asp Ala Gln Glu Leu Thr Glu Thr Ala Leu Lys Ser Lys Tyr
        955                 960                 965

gct tca aga tta agt ttc tct aaa aat gaa caa atc agc att ttt gtc 3761
Ala Ser Arg Leu Ser Phe Ser Lys Asn Glu Gln Ile Ser Ile Phe Val
    970                 975                 980

acc ggt gct act ggg ttc tta ggt tca ttc att att gct gat tta ttg 3809
Thr Gly Ala Thr Gly Phe Leu Gly Ser Phe Ile Ile Ala Asp Leu Leu
985                 990                 995                 1000

aac cgt aaa tca aac atc aaa atc ttt gct cat gtt cgt gcc gct 3854
Asn Arg Lys Ser Asn Ile Lys Ile Phe Ala His Val Arg Ala Ala
                1005                1010                1015

gat aaa gtt aaa ggt ctc gaa aga ttg aaa aaa gct ggt tta acc 3899
Asp Lys Val Lys Gly Leu Glu Arg Leu Lys Lys Ala Gly Leu Thr
                1020                1025                1030

tac ggt aat tgg aaa gat gaa ttt gcc tca tca att gaa gtt gtt 3944
Tyr Gly Asn Trp Lys Asp Glu Phe Ala Ser Ser Ile Glu Val Val
                1035                1040                1045

tta ggt gat tta tca aaa gaa caa ttt ggt tta gat ttc caa gct 3989
Leu Gly Asp Leu Ser Lys Glu Gln Phe Gly Leu Asp Phe Gln Ala
                1050                1055                1060

tgg ggt aac ttg act gaa aca att gat gtt att att cat aat ggt 4034
Trp Gly Asn Leu Thr Glu Thr Ile Asp Val Ile Ile His Asn Gly
                1065                1070                1075

gct tta gtt cac tgg gtg tat cca tat gat aaa tta cgt gat gcc 4079
Ala Leu Val His Trp Val Tyr Pro Tyr Asp Lys Leu Arg Asp Ala
                1080                1085                1090

aat gtt atc ggt act atc aac gtt ttg aat tta gct gct tct ggt 4124
Asn Val Ile Gly Thr Ile Asn Val Leu Asn Leu Ala Ala Ser Gly
                1095                1100                1105

aag gct aag tat ttc tca ttt gtt tct tca aca tca gct tta gat 4169
Lys Ala Lys Tyr Phe Ser Phe Val Ser Ser Thr Ser Ala Leu Asp
                1110                1115                1120

act caa cat ttc gtt gaa tta tct gat aaa tta ctt tct gaa ggt 4214
Thr Gln His Phe Val Glu Leu Ser Asp Lys Leu Leu Ser Glu Gly
                1125                1130                1135

aaa gat ggt att cca  gaa tct gat gat tta  caa ggt tct gct tac        4259
Lys Asp Gly Ile Pro  Glu Ser Asp Asp Leu  Gln Gly Ser Ala Tyr
                1140                 1145                 1150

ggt tta ggt tca ggt  tac ggt caa agt aaa  tgg gct gct gaa tat        4304
Gly Leu Gly Ser Gly  Tyr Gly Gln Ser Lys  Trp Ala Ala Glu Tyr
                1155                 1160                 1165

att atc cgt cgt gct  ggt gaa cgt ggt ttg  aaa ggt gct att att        4349
Ile Ile Arg Arg Ala  Gly Glu Arg Gly Leu  Lys Gly Ala Ile Ile
                1170                 1175                 1180

aga cca ggt tat gtt  act ggt gaa act aat  act ggt tct tca aac        4394
Arg Pro Gly Tyr Val  Thr Gly Glu Thr Asn  Thr Gly Ser Ser Asn
                1185                 1190                 1195

act gat gat ttc tta  tta cgt atg ttg aaa  ggt tgt gct gaa tta        4439
Thr Asp Asp Phe Leu  Leu Arg Met Leu Lys  Gly Cys Ala Glu Leu
                1200                 1205                 1210

ggt aaa tat cca aac  att aat aac acc atc  aat atg gtc cct gtt        4484
Gly Lys Tyr Pro Asn  Ile Asn Asn Thr Ile  Asn Met Val Pro Val
                1215                 1220                 1225

gat cat gtt gca aga  gtt gtt gtt gca gct  gcc tta cat cca cca        4529
Asp His Val Ala Arg  Val Val Val Ala Ala  Ala Leu His Pro Pro
                1230                 1235                 1240

aaa tca gaa gaa tta  gct gtt gtt caa gtt  act ggt cat cca cgt        4574
Lys Ser Glu Glu Leu  Ala Val Val Gln Val  Thr Gly His Pro Arg
                1245                 1250                 1255

atc aca ttt att caa  ttt gtt ggt gca ttg  aaa aaa tat ggt tat        4619
Ile Thr Phe Ile Gln  Phe Val Gly Ala Leu  Lys Lys Tyr Gly Tyr
                1260                 1265                 1270

gaa att gaa tta gaa  gat tat gtt act tgg  aaa tca tca tta gaa        4664
Glu Ile Glu Leu Glu  Asp Tyr Val Thr Trp  Lys Ser Ser Leu Glu
                1275                 1280                 1285

aaa ttt gtt ttg gaa  ggt act caa gaa agt  gca ctt tat cca tta        4709
Lys Phe Val Leu Glu  Gly Thr Gln Glu Ser  Ala Leu Tyr Pro Leu
                1290                 1295                 1300

tta cat ttt gtt tta  gat gat tta cca caa  gat act aaa gct cca        4754
Leu His Phe Val Leu  Asp Asp Leu Pro Gln  Asp Thr Lys Ala Pro
                1305                 1310                 1315

gaa tta gat gat gtt  aat gct cgt act tca  ttg aaa gca gat gct        4799
Glu Leu Asp Asp Val  Asn Ala Arg Thr Ser  Leu Lys Ala Asp Ala
                1320                 1325                 1330

gaa tgg act ggc gtt  gat gcg tca gct ggt  aga ggt att gat gaa        4844
Glu Trp Thr Gly Val  Asp Ala Ser Ala Gly  Arg Gly Ile Asp Glu
                1335                 1340                 1345

caa caa tta agc gtt  tac gtt gca tac ttg  att aaa gtt ggt ttc        4889
Gln Gln Leu Ser Val  Tyr Val Ala Tyr Leu  Ile Lys Val Gly Phe
                1350                 1355                 1360

tta cca aaa cca tca  aaa gct ggc gaa cca  tta cca gaa gtt aag        4934

Leu Pro Lys Pro Ser Lys Ala Gly Glu Pro Leu Pro Glu Val Lys
                1365                1370                1375

tta act aaa gaa caa ctc gat ttg att aca tct ggt gct ggt tct 4979
Leu Thr Lys Glu Gln Leu Asp Leu Ile Thr Ser Gly Ala Gly Ser
                1380                1385                1390

cgt ggt agt tca aat tag atagatttga agtaaaaaa aaacataaat 5027
Arg Gly Ser Ser Asn
                1395

tacgtctgta catatagtta aaatattcgt taaataaatt gatttataga ttttgttaca 5087

attctgtaga gtttgttatt tattaaaccg aatatattat atgtgtatta acctttgtac 5147

atatgaaatc tctaattcat aaataaaaac tgtaaatcat caggggttaa tctattaatt 5207

gcaccatcat catggttaat ggtagcatga atcatatttg ccttcttatc ttgtaattct 5267

ataattcttg attctatact atcttcaata caaaatcttg taattttaac aggtctaaat 5327

tgaccaattc tatgaactct atcaccactt tgccattcaa cagatggatt ccaccaagga 5387

tccattaaga aaacttgtga agcttcacat aaatttaaag caacaccacc agctttcaaa 5447

ctgaccaaga aaacttcaac atttgtattt tccataaaat gacgaattgt attatctctt 5507

tgaataggtg acattgaacc ctgtaattta acagtttgaa aacctgctct tttcaatctc 5567

cattcaatta aatctaacat acttgtaaat tgtgaaaaca caatagattt aatagtttgt 5627

ctatcacttc ttaatttata aagttcttct actaaagctt caatctttgt agatgacctc 5687

cattcaccac ccattttaat tctattaaca attgatgtta ttattcataa tggtgcttta 5747

gttcactggg tgtatccata tgataaatta cgtgatgcca atgttatcgg tactatcaac 5807

gttttgaatt tagctgcttc tggtaaggct aagtatttct catttgtttc ttcaacatca 5867

gctttagata ctcaacattt cgttgaatta tct 5900

<210> 4
<211> 1395
<212> PRT
<213> Pichia ciferrii

<400> 4

Met Ser Ser Asp Leu Gln His Trp Ala Gln Ile Leu Ser Ser Pro Thr
1               5                   10                  15

Leu Ser Val Leu Pro His Asp Phe Ile Arg Pro Ser Asn Asn Lys Val
            20                  25                  30

Val Glu Ala Lys His Asn Phe Ser Ile Pro Ala Lys Ala Leu Ser Gln
        35                  40                  45

Ser Ile Ile Ser Lys Val Glu Gly Ser Asn Glu Phe Ile Ile Gly Leu
    50                  55                  60

Ser Val Phe Ala Val Leu Ile Tyr Arg Leu Thr Gly Asp Asp Asp Ile
65                  70                  75                  80

Val Ile Ser Thr Asp Ser Pro Asn Gly Gly Lys Glu Phe Val Ile Arg
                85                  90                  95

Leu Thr Ile Ser Pro Thr Val Ser Phe Val Glu Leu Val Glu Gln Ile
            100                 105                 110

Thr Lys Leu Tyr Asn Asp Asn Val Asn Arg Ala Val Asp Tyr Asp Glu
        115                 120                 125

Leu Ser Ala Leu Ile Gln Lys Glu Asn Gly Tyr Glu Thr Lys Pro Ser
    130                 135                 140

Leu Phe Arg Leu Ser Phe Gln Tyr Gly Asn Ala His Gln Gln Leu Ser
145                 150                 155                 160

Glu Tyr Gly Ser Ile Arg Asp Leu Ser Ile Phe Val Asn Asp Asp Ser
                165                 170                 175

Tyr Asp Val Tyr Phe Asn Ala Leu Leu Tyr Lys Asp Asp Arg Ile Ser
            180                 185                 190

Ile Leu Ala Glu Gln Phe Asn Gln Phe Leu Glu Ala Val Ala Gln Asp
        195                 200                 205

Ser Ser Leu Gln Val Thr Lys Ile Asn Leu Ile Thr Lys Ser Gln Lys
    210                 215                 220

Gln Ile Leu Pro Asp Pro Thr Thr Asp Leu Asp Trp Ser Gly Tyr Arg
225                 230                 235                 240

Gly Ala Ile His Asp Ile Phe Gln Glu Asn Ala Ala Lys Phe Pro Glu
                245                 250                 255

Arg Thr Cys Val Val Glu Thr Pro Ser Phe Leu Thr Pro Glu Lys Gln
            260                 265                 270

Ala Arg Val Phe Thr Tyr Lys His Ile Asn Glu Ala Ser Asn Ile Val
        275                 280                 285

Gly Asn Tyr Leu Val Lys Thr Gly Ile Lys Arg Gly Asp Val Val Met

290                 295                 300

Ile Tyr Ser Ser Arg Gly Val Asp Leu Met Val Ser Val Leu Gly Val
305                 310                 315                 320

Leu Lys Ala Gly Ala Thr Phe Ser Val Ile Asp Pro Ala Tyr Pro Pro
                325                 330                 335

Ala Arg Gln Asn Ile Tyr Leu Gln Val Ala Lys Pro Lys Gly Leu Ile
            340                 345                 350

Val Ile Lys Lys Ala Gly Val Leu Asp Glu Leu Val Glu Asn Tyr Ile
        355                 360                 365

Lys Asp Glu Leu Asp Val Val Ser Arg Val Pro Gln Ile Glu Leu Gln
    370                 375                 380

Asp Asp Gly Ser Val Leu Gly Gly Lys Asn Leu Glu Gly Glu Asp Val
385                 390                 395                 400

Leu Ala Gly Val Ser Thr Ser Ser Thr Gly Val Leu Val Gly Pro Asp
                405                 410                 415

Ser Asn Pro Thr Leu Ser Phe Thr Ser Gly Ser Glu Gly Leu Pro Lys
            420                 425                 430

Gly Val Leu Gly Arg His Phe Ser Leu Ala Tyr Tyr Phe Asp Trp Met
        435                 440                 445

Ser Lys Thr Phe Lys Leu Asn Ser Asp Asp Lys Phe Thr Met Leu Ser
    450                 455                 460

Gly Ile Ala His Asp Pro Ile Gln Arg Asp Met Phe Thr Pro Ile Phe
465                 470                 475                 480

Leu Gly Ala Gln Leu Leu Val Pro Thr Ala Asp Asp Ile Gly Thr Pro
                485                 490                 495

Gly Lys Leu Ala Glu Trp Met Gly Lys Tyr Gly Ala Thr Val Thr His
            500                 505                 510

Leu Thr Pro Ala Met Gly Gln Leu Leu Ser Ala Gln Ala Thr Ser Glu
        515                 520                 525

Ile Pro Ser Leu His His Ala Phe Phe Val Gly Asp Ile Leu Thr Lys
    530                 535                 540

Arg Asp Cys Leu Arg Leu Gln Thr Leu Ala Lys Asn Val Phe Ile Val
545                 550                 555                 560

Asn Met Tyr Gly Thr Thr Glu Thr Gln Arg Ala Val Ser Tyr Phe Glu
                565                 570                 575

Val Ala Ser Arg Ala His Asp Ser Thr Phe Leu Glu Ser Gln Lys Asp
            580                 585                 590

Val Met Pro Ala Gly Lys Gly Met Leu Asn Val Gln Leu Leu Val Val
        595                 600                 605

Asn Arg Asn Asp Thr Ser Gln Thr Cys Gly Val Gly Glu Val Gly Glu
    610                 615                 620

Ile Tyr Val Arg Ala Ala Gly Leu Ala Glu Gly Tyr Arg Gly Leu Pro
625                 630                 635                 640

Glu Leu Asn Glu Gln Lys Phe Val Lys Asn Trp Tyr Val Asp Ala Gln
                645                 650                 655

Gln Trp Ile Asp Gln Asp Asn Gln Lys Ser Gln Asn Glu Pro Trp Arg
            660                 665                 670

Glu Phe Trp Lys Gly Pro Arg Asp Arg Leu Tyr Arg Thr Gly Asp Leu
        675                 680                 685

Gly Arg Tyr Leu Pro Asp Gly Asn Thr Glu Cys Cys Gly Arg Ala Asp
    690                 695                 700

Asp Gln Val Lys Ile Arg Gly Phe Arg Ile Glu Leu Gly Glu Ile Asp
705                 710                 715                 720

Thr Asn Ile Ser Gln His Pro Leu Val Arg Glu Asn Val Thr Leu Val
                725                 730                 735

Arg Lys Asp Ala Asn Asn Glu Pro Thr Leu Ile Ser Tyr Ile Val Pro
            740                 745                 750

Lys Thr Glu Val Ala Asp Leu Glu Asn Phe Lys Ser Ser Val Glu Glu
        755                 760                 765

Leu Glu Asn Lys Asp Asp His Val Val Ser Gly Leu Val Gly Tyr Arg
    770                 775                 780

Leu Leu Ile Lys Asp Ile Lys Ala Phe Leu Lys Lys Arg Leu Ala Ser
785                 790                 795                 800

Tyr Ala Ile Pro Thr Ile Ile Val Pro Phe His Lys Leu Pro Leu Asn
                805                 810                 815

Pro Asn Gly Lys Val Asp Lys Pro Lys Leu Pro Phe Pro Thr Thr Ala
            820                 825                 830

Gln Leu Glu Ala Val Ala Lys His Ser Ser Ala Asp Gln Asp Asp Ser
        835                 840                 845

Gln Phe Ser Glu Thr Glu Gly Lys Val Arg Asp Val Trp Leu Ser Val
    850                 855                 860

Leu Pro Gln Arg Pro Ser Thr Ile Ser Pro Glu Asp Ser Phe Phe Asp
865                 870                 875                 880

Leu Gly Gly His Ser Ile Leu Ala Thr Arg Met Ile Phe Glu Leu Arg
                885                 890                 895

Lys Gln Leu Val Ile Asp Leu Pro Leu Gly Thr Ile Phe Lys Phe Pro
            900                 905                 910

Thr Ile Lys Ala Phe Ala Lys Glu Ile Glu Arg Val Gln Lys Gly Asp
        915                 920                 925

Asp Val Ala Leu Pro Ala Thr Ser Gly Ala Glu Asn Thr Asp Glu Asp
    930                 935                 940

Leu Ala Asn Lys Pro Ala Glu Tyr Tyr Lys Asp Ala Gln Glu Leu Thr
945                 950                 955                 960

Glu Thr Ala Leu Lys Ser Lys Tyr Ala Ser Arg Leu Ser Phe Ser Lys
                965                 970                 975

Asn Glu Gln Ile Ser Ile Phe Val Thr Gly Ala Thr Gly Phe Leu Gly
            980                 985                 990

Ser Phe Ile Ile Ala Asp Leu Leu  Asn Arg Lys Ser Asn  Ile Lys Ile
        995                 1000                1005

Phe Ala  His Val Arg Ala Ala  Asp Lys Val Lys Gly  Leu Glu Arg
    1010                1015                1020

Leu Lys Lys Ala Gly Leu Thr Tyr Gly Asn Trp Lys Asp Glu Phe
    1025                1030                1035

Ala Ser Ser Ile Glu Val Val Leu Gly Asp Leu Ser Lys Glu Gln
    1040                1045                1050

Phe Gly Leu Asp Phe Gln Ala Trp Gly Asn Leu Thr Glu Thr Ile
    1055                1060                1065

Asp Val Ile Ile His Asn Gly Ala Leu Val His Trp Val Tyr Pro
    1070                1075                1080

Tyr Asp Lys Leu Arg Asp Ala Asn Val Ile Gly Thr Ile Asn Val
    1085                1090                1095

Leu Asn Leu Ala Ala Ser Gly Lys Ala Lys Tyr Phe Ser Phe Val
    1100                1105                1110

Ser Ser Thr Ser Ala Leu Asp Thr Gln His Phe Val Glu Leu Ser
    1115                1120                1125

Asp Lys Leu Leu Ser Glu Gly Lys Asp Gly Ile Pro Glu Ser Asp
    1130                1135                1140

Asp Leu Gln Gly Ser Ala Tyr Gly Leu Gly Ser Gly Tyr Gly Gln
    1145                1150                1155

Ser Lys Trp Ala Ala Glu Tyr Ile Ile Arg Arg Ala Gly Glu Arg
    1160                1165                1170

Gly Leu Lys Gly Ala Ile Ile Arg Pro Gly Tyr Val Thr Gly Glu
    1175                1180                1185

Thr Asn Thr Gly Ser Ser Asn Thr Asp Asp Phe Leu Leu Arg Met
    1190                1195                1200

Leu Lys Gly Cys Ala Glu Leu Gly Lys Tyr Pro Asn Ile Asn Asn
    1205                1210                1215

Thr Ile Asn Met Val Pro Val Asp His Val Ala Arg Val Val Val
    1220                1225                1230

Ala Ala Ala Leu His Pro Pro Lys Ser Glu Glu Leu Ala Val Val
    1235                1240                1245

Gln Val Thr Gly His Pro Arg Ile Thr Phe Ile Gln Phe Val Gly

1250                1255                1260

Ala Leu Lys Lys Tyr Gly Tyr Glu Ile Glu Leu Glu Asp Tyr Val
    1265                1270                1275

Thr Trp Lys Ser Ser Leu Glu Lys Phe Val Leu Glu Gly Thr Gln
    1280                1285                1290

Glu Ser Ala Leu Tyr Pro Leu Leu His Phe Val Leu Asp Asp Leu
    1295                1300                1305

Pro Gln Asp Thr Lys Ala Pro Glu Leu Asp Asp Val Asn Ala Arg
    1310                1315                1320

Thr Ser Leu Lys Ala Asp Ala Glu Trp Thr Gly Val Asp Ala Ser
    1325                1330                1335

Ala Gly Arg Gly Ile Asp Glu Gln Gln Leu Ser Val Tyr Val Ala
    1340                1345                1350

Tyr Leu Ile Lys Val Gly Phe Leu Pro Lys Pro Ser Lys Ala Gly
    1355                1360                1365

Glu Pro Leu Pro Glu Val Lys Leu Thr Lys Glu Gln Leu Asp Leu
    1370                1375                1380

Ile Thr Ser Gly Ala Gly Ser Arg Gly Ser Ser Asn
    1385                1390                1395

## Description

The industrial yeast *Pichia ciferrii* is known for its unique ability to produce high levels of sphingoid bases and derivatives thereof, mainly C18-phytosphingosine and acetylated derivatives thereof (Wickerham and Stodola, Formation of extracellular sphingolipids by microorganisms. I. Tetraacetylphytosphingosine from Hansenula ciferri. J Bacteriol. 1960 Oct;80:484-91). These can be extracted and chemically converted into corresponding ceramides (see e.g. WO 93/20038). Both sphingoid bases and ceramides are specialty chemicals of considerable commercial significance for food, pharmaceutical and cosmetic applications.

Consequently, it would be desirable to design *Pichia ciferrii* strains capable of efficiently producing sphingolipids other than phytosphingosine, e.g. sphingosine. However, sphingolipids are produced via a complex biosynthetic pathway, which is composed of multiple enzymatic steps required for the biosynthesis of a given sphingolipid, but also contains several branching points potentially being detrimental for the production of a given sphingolipid. Therefore, engineering the sphingolipid biosynthetic pathway, but also other biosynthetic pathways, in *Pichia ciferrii* will require a multitude of rational modifications of the genetic information naturally contained in this industrial yeast. The rational modification of genetic material requires the availability of suitable transformation systems. However, only one such system has so far been described for *Pichia ciferrii* (Bae et al., Integrative transformation system for the metabolic engineering of the sphingoid base-producing yeast Pichia ciferrii. Appl Environ Microbiol. 2003. 69:812-9; WO 99/57279). It relies on a modified ribosomal protein L41 which confers resistance against cycloheximide. Therefore, its applicability is limited, as it allows only for a single selection round after transformation with recombinant DNA.

It would be highly desirable to avail of a transformation system which can be repeatedly used and which allows for multiple events of genetic engineering, allowing *Pichia ciferrii* to be genetically modified in a rational manner, with the purpose to use this yeast for the production of low-molecular-weight compounds, such as sphingolipids, but also for the production of macromolecules, such as peptides or proteins.

In a first aspect, the present invention provides *Pichia ciferrii* cells defective in a biosynthetic pathway. The biosynthetic pathway may be a pathway for biosynthesis of an amino acid, a nucleotide and/or a vitamin. More preferably, the biosynthetic pathway is a pathway for biosynthesis of uracil, adenine, tryptophan, lysine and/or methionine. Most preferably, the biosynthetic pathway is a pathway for biosynthesis of uracil and/or lysine.

According to the invention, "defective in a biosynthetic pathway" means that a cell possessing that feature is not capable to synthesise the metabolite that is the product of that biosynthetic pathway. The *Pichia ciferrii* cells thereby may be defective in one or more biosynthetic pathways.

The *Pichia ciferrii* cells defective in a biosynthetic pathway were surprisingly obtained by simple selection of spontaneous mutants capable of growing in the presence of an otherwise toxic metabolite analogue.

The method to prepare the *Pichia ciferrii* cells defective in a biosynthetic pathway comprises culturing a population of *Pichia ciferrii* cells in the presence of a toxic metabolite analogue under conditions wherein the majority of cells is not capable to grow, and isolating clones that are able to grow.

If necessary, the amount of mutants may be increased by subjecting the population of *Pichia ciferrii* cells to UV or chemical mutagenesis prior to the culturing in the presence of the toxic metabolite analogue.

A suitable concentration of the toxic metabolite analogue typically lies around the minimal inhibitory concentration (MIC value) found for the particular toxic metabolite analogue when cultured with the *Pichia ciferrii* cells. This concentration typically may be between, for instance, 1 and 10 mg/ml.

The toxic metabolite analogue to be used will depend on the biosynthetic pathway to be made defective. It may be 5-fluoroorotic acid, α-aminoadipic acid, 5-fluoroanthranilic acid, ureidosuccinic acid and/or methyl mercury, providing *Pichia ciferrii* cells that are defective in the metabolic pathway for uracil, lysine, tryptophan and/or methionine respectively. Preferably, the toxic metabolite analogue is 5-fluoroorotic acid and/or α-aminoadipic acid. Resistance of the provided *Pichia ciferrii* cells against these metabolite analogues is due to spontaneous (or induced if classical mutagenesis techniques are applied) loss-of-function mutations in the structural genes encoding those biosynthetic enzymes which would otherwise metabolize the metabolite analogues, resulting in toxicity to the cells.

Alternatively, mutant strains with loss-of-function mutations in a structural gene encoding a biosynthetic enzyme can be isolated by other means of enrichment of spontaneous auxotrophic mutants. For instance, enrichment of such spontaneous auxotrophic mutants can be performed by cultivation of a population of cells in a minimal medium containing substances which kill growing cells, such as nystatin. Consequently, cells with mutations resulting in an auxotrophic phenotype can not grow, survive and are thereby enriched. Auxotrophic mutants can subsequently be identified by testing their growth requirements.

Thus, the biosynthetic enzymes inactivated by loss-of-function mutations may be dihydroorotate dehydrogenase, orotidine-5'-phosphate decarboxylase, orotate phosphoribosyltransferase, tryptophan synthase, homoserine O-acetyltransferase, sulfite reductase, O-acetylhomoserine O-acetylserine sulfydrylase, and/or α-aminoadipate reductase. Preferably, the biosynthetic enzymes inactivated by loss-of-function mutations are orotidine-5'-phosphate decarboxylase or α-aminoadipate reductase. Inactivation of α-aminoadipate reductase may be due to loss-of-function mutations in the gene encoding α-aminoadipate reductase *(LYS2)* or may alternatively caused by loss-of-function mutations in the gene encoding α-aminoadipate reductase phosphopantetheinyl transferase *(LYS5).* α-Aminoadipate reductase phosphopantetheinyl transferase is required for post-translational modification of α-aminoadipate reductase, which is essential for α-aminoadipate reductase activity.

The *Pichia ciferrii* mutants defective in a biosynthetic pathway appear to display a stable auxotrophic phenotype, which means that they do not revert to a prototrophy upon prolonged culturing under non-selective conditions, or only show a low reversion frequency.

It is very surprising that spontaneous *Pichia ciferrii* mutants defective in a biosynthetic pathway and possessing a stable auxotrophic phenotype could be obtained. Successful selection of spontaneous or even UV-induced auxotrophic mutants has to our knowledge only been described for haploid yeast species such as *Saccharomyces cerevisiae, Candida utilis* (W09814600) and *Candida lusitanae* (Young et al., A STE12 homolog is required for mating but dispensable for filamentation in Candida lusitaniae. Genetics. 2000. 155:17-29; Francois et al., Development of an integrative transformation system for the opportunistic pathogenic yeast Candida lusitaniae using URA3 as a selection marker. Yeast. 2004. 21:95-106), but not for diploid yeast species such as *Pichia ciferrii* (Wickerham and Burton, 1962, Bacteriol Rev. 26:382-397). Attempts to obtain spontaneous auxotrophic mutants of diploid yeast cells have repeatedly been described, but reportedly failed, even if the diploid yeast cells were mutagenized prior to selection for auxotrophic phenotypes (e.g. Wellington and Rustchenko, 5-Fluoroorotic acid induces chromosome alterations in Candida albicans. Yeast. 2005 Jan 15;22(1):57-70.).

In fact, though the *URA3* marker is used as selection marker in the diploid yeast *Candida albicans,* the required homozygous *ura3*/*ura3* strains had to be created by laborious manipulations such as two rounds of targeted inactivation (Gorman et al., Repeated use of GAL1 for gene disruption in Candida albicans. Genetics. 1991. 129:19-24; Fonzi and Irwin, Isogenic strain construction and gene mapping in Candida albicans. Genetics. 1993. 134:717-28; Morschhäuser et al. Sequential gene disruption in Candida albicans by FLP-mediated site-specific recombination. Mol Microbiol. 32:547-56.) or by targeted inactivation via cotransformation of the deletion construct and a replicative plasmid containing the selection marker (Kelly et al., One-step gene disruption by cotransformation to isolate double auxotrophs in Candida albicans. Mol Gen Genet. 1988. 214:24-31.).

These laborious manipulations would not be easily applicable to *Pichia ciferrii.* They would either require the availability of two different dominant selection markers (sequential disruption of both alleles), conditional promoters functional in *Pichia ciferrii* (FLP recombinase-mediated marker rescue), or vectors capable of autonomous replication (co-transformation). However, none of these tools is available for *Pichia ciferrii.*

The *Pichia ciferrii* cells of the first aspect defective in a biosynthetic pathway may be host cells in a transformation system further comprising a polynucleotide comprising a functional gene that complements the defect in the biosynthetic pathway in which the *Pichia ciferrii* cells are defective.

In a second aspect there is provided a method of transforming a *Pichia ciferrii* cell of the first aspect having a defective biosynthetic pathway, comprising transforming the cell with a polynucleotide containing a functional gene that complements the defect in the biosynthetic pathway. Typically, the complementing functional gene encodes a functional enzyme of the biosynthetic pathway. The complementing functional gene may be any functional gene providing complementation of the defect in the biosynthetic pathway. It may be from any suitable origin, i.e. from bacterial, yeast, fungal, plant or animal species, but preferably is from a yeast. Most preferred is the use of a functional gene originating from *Pichia ciferrii.*

In a third aspect of the present invention there is provided a novel polynucleotide for use in a method of the previous aspect, wherein the polynucleotide contains a functional gene that complements the defect in said biosynthetic pathway in which the host cell is defective. The polynucleotide preferably is capable of integration into the genome of the *Pichia ciferrii* host cell.

In one embodiment of the invention, the complementing functional gene has a nucleotide sequence encoding a polypeptide with an amino acid sequence of SEQ ID NO:2, an amino acid sequence of SEQ ID NO:4, an amino acid sequence having a degree of identity of at least 91%, preferably at least 95%, to the amino acid sequence of SEQ ID NO:2 and/or an amino acid sequence having a degree of identity of at least 70%, preferably at least 80%, more preferably at least 90%, most preferably at least 95%, to the amino acid sequence of SEQ ID NO:4. Preferred functional genes have a nucleotide sequence according to SEQ ID NO:1, SEQ ID NO:3 and/or a sequence degenerate thereto.

The polynucleotide of this aspect may be in the form of a vector that may conveniently be subjected to recombinant DNA procedures and can be successfully introduced into *Pichia ciferrii* by transformation. The vector may be an autonomously replicating vector, i.e. a vector which exists as an extra-chromosomal entity, the replication of which is independent of chromosomal replication, e.g. a plasmid, cosmid, virus or phage vector, usually provided with an origin of replication. Preferably, the vector may be one which, when introduced into a host cell, is integrated into the host cell genome and replicated together with the chromosome(s) into which it has been integrated.

The vector may contain an expression cassette wherein the complementing functional gene is operably linked to regulatory sequences that are capable of providing for the expression of the encoded polypeptide by the *Pichia ciferrii* cells. Preferably, the expression cassette is flanked at its 5' and 3' sides by direct repeats of DNA. After the expression cassette flanked by direct repeats of DNA has been introduced into *Pichia ciferrii* cells by transformation, homologous recombination between the direct repeats of DNA may occur, leading to deletion of the selection marker contained in the expression cassette. This enables the use of *Pichia ciferrii* for sequential rounds of transformation. Cells that have lost the selection marker may be selected for by using the toxic metabolite analogue as described above. The direct repeats of DNA flanking the expression cassette typically have a length of at least 20 bp, preferably at least 100 bp, and have a degree of identity of at least 80% over their whole length. They may be derived from *Pichia ciferrii* or from another yeast or any other suitable source. Alternatively, they may be synthetically produced polynucleotides.

In a fourth aspect, the present invention provides novel polypeptides that are able to complement a defect in a biosynthetic pathway of *Pichia ciferrii.*

In one embodiment a polypeptide is provided that complements a defect in the uracil biosynthetic pathway and has an amino acid sequence according to SEQ ID NO: 2 or an amino acid sequence displaying a degree of identity of at least 91 %, preferably at least 95%, to the amino acid sequence of SEQ ID NO:2. In another embodiment a polypeptide is provided that complements a defect in the lysine biosynthetic pathway and has an amino acid sequence according to SEQ ID NO: 4 or an amino acid sequence having a degree of identity of at least 70%, preferably at least 80%, more preferably at least 90%, most preferably at least 95%, to the amino acid sequence of SEQ ID NO: 4.

In a further aspect the invention provides a method for preparing a compound of interest using *Pichia ciferrii* cells defective in a biosynthetic pathway. The method comprises transforming *Pichia ciferrii* cells of the first aspect of this invention, i.e. defective in a biosynthetic pathway, with a polynucleotide of the third aspect (the selection marker) and a polynucleotide comprising a DNA sequence of interest, selecting transformed cells for prototrophy, culturing transformed cells under conditions conducive to production of the compound of interest, and, optionally, recovering the compound of interest.

The polynucleotides comprising the selection marker and the DNA sequence of interest may be contained in one DNA molecule or may be separate DNA molecules.

If the compound of interest is a polypeptide, the DNA sequence of interest typically encodes the polypeptide of interest. If the polypeptide is produced as a secreted protein, the nucleotide sequence encoding a mature form of the polypeptide in the expression construct is operably linked to a nucleotide sequence encoding a signal peptide.

If the compound of interest is a metabolite, e.g. a sphingoid base or other sphingolipids, the DNA sequence of interest may be a sequence suitable for metabolic engineering. For instance, the DNA sequence of interest may encode an enzyme of the metabolic pathway leading to production of the compound of interest. It is also possible that the DNA sequence of interest is intended to inactivate a gene encoding an enzyme activity that is undesirable for production of the compound of interest.

### Brief description of the Figures

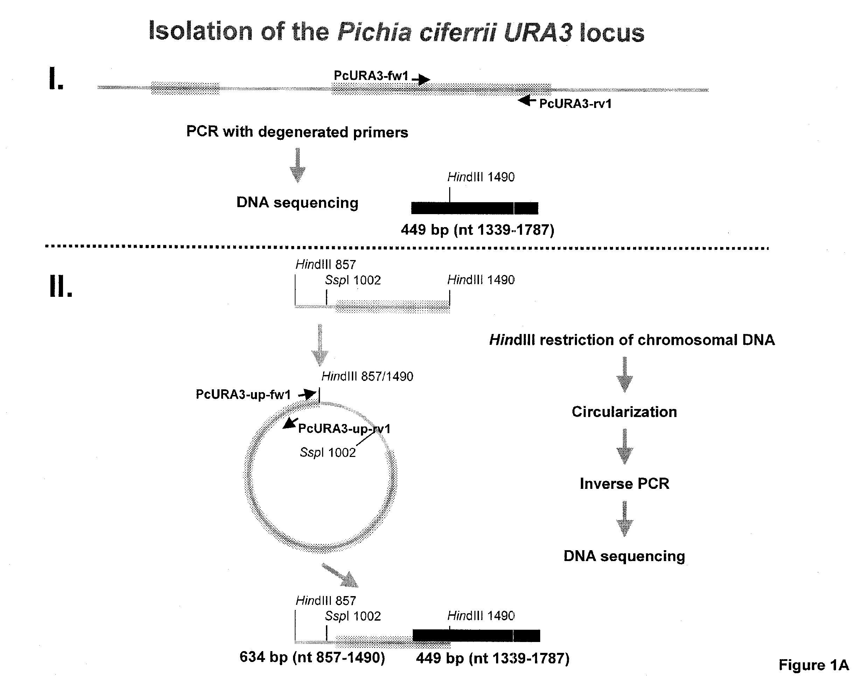

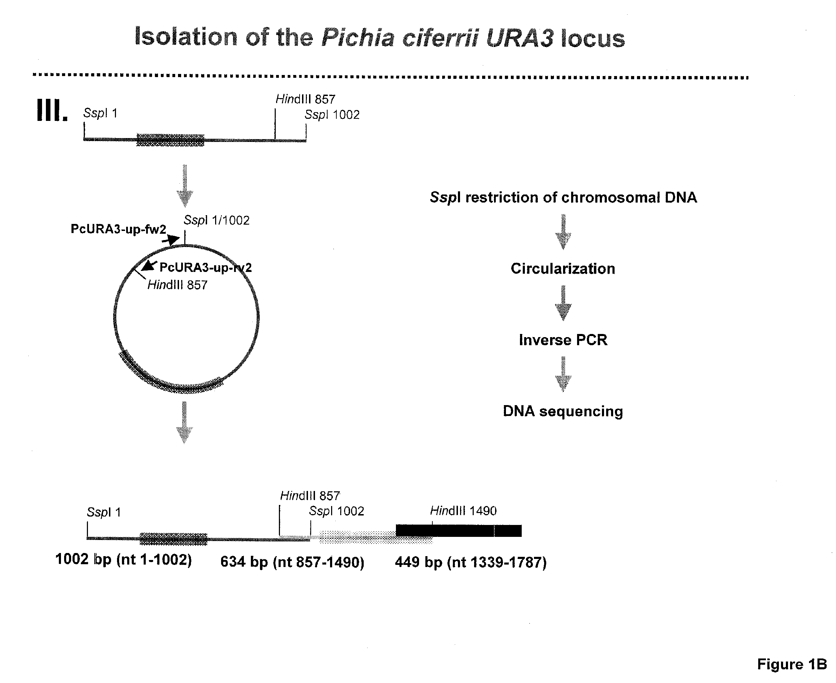

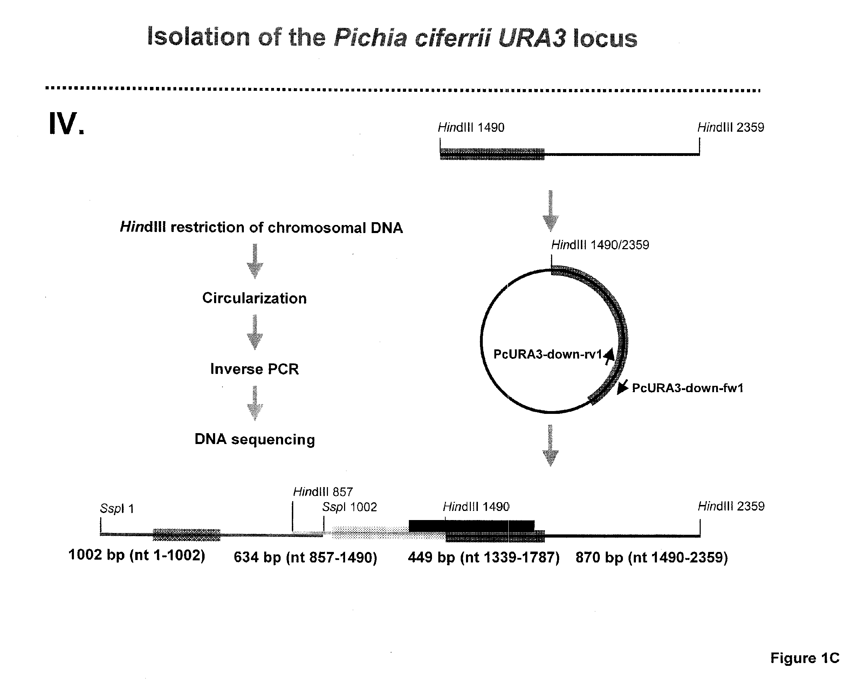

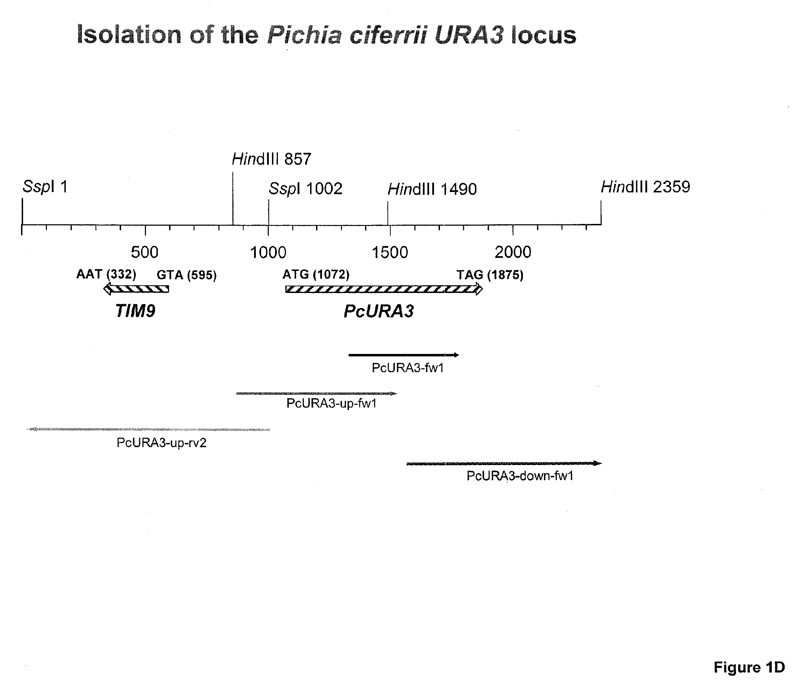

**Fig. 1** schematically describes the four-step procedure resulting in the isolation of the entire *Pichia ciferrii URA3* locus.
   Amplification of an internal part of *PcURA3* (I.) was followed by three rounds of inverse PCR (II. to IV.) Oligonucleotides used in the individual steps are indicated and sequence representations in different shadings show the portions of the *PcURA3* locus whose DNA sequence were determined in the individual steps. Restriction sites relevant for the experimental procedures are also indicated.

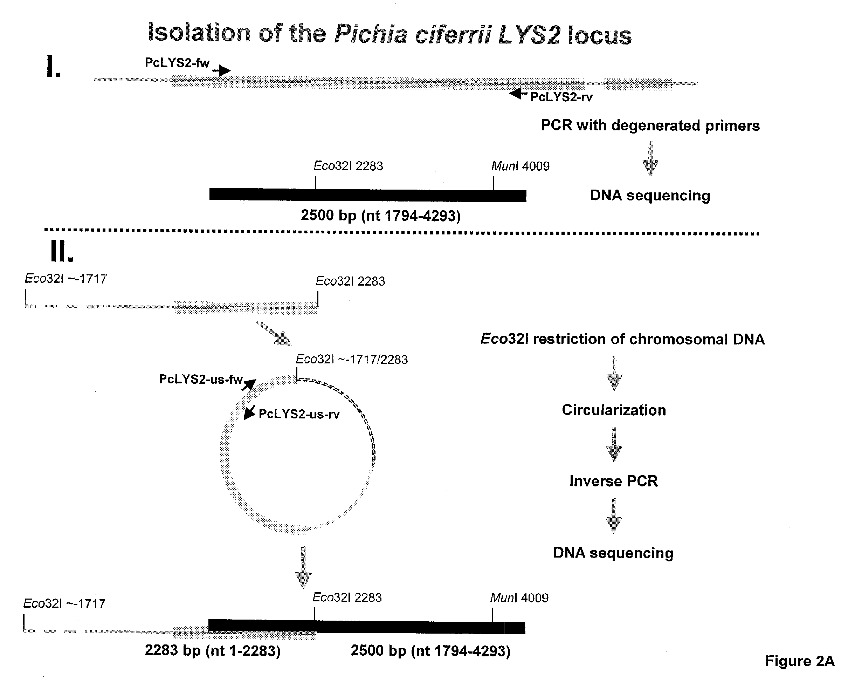

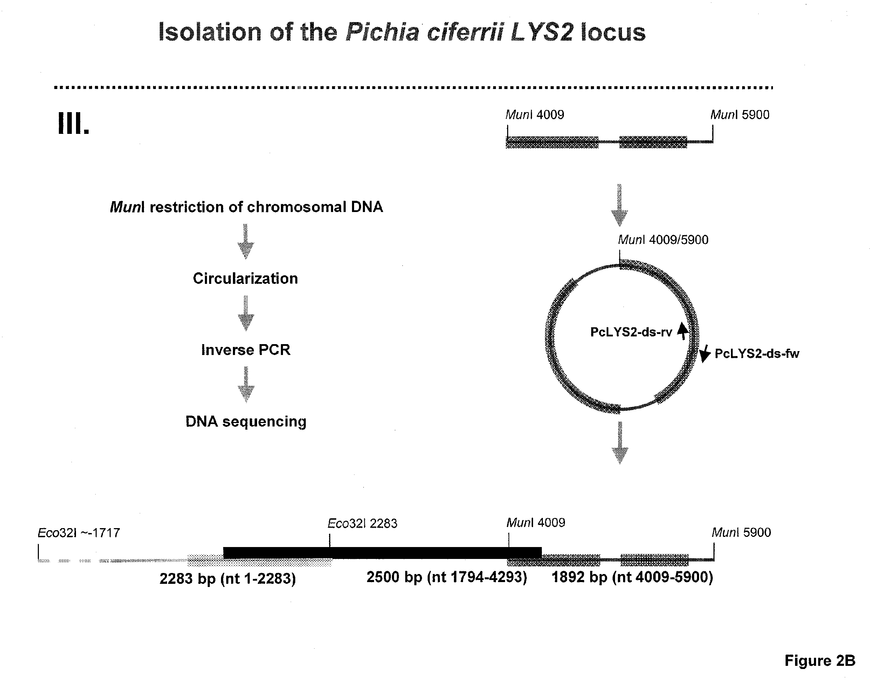

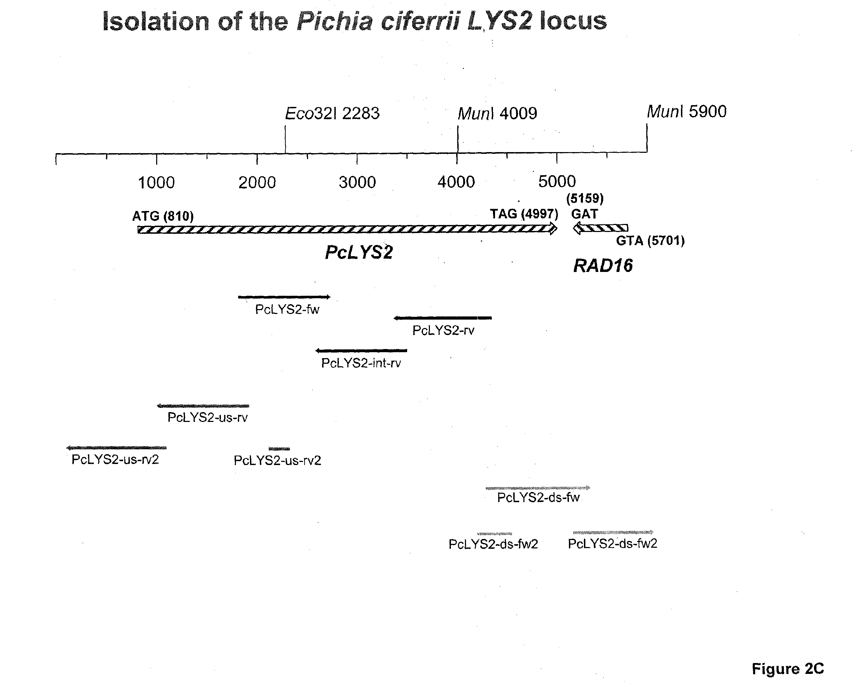

**Fig. 2** schematically describes the three-step procedure resulting in the isolation of the entire *Pichia ciferrii LYS2* locus.
   Amplification of an internal part of *PcLYS2* (I.) was followed by two rounds of inverse PCR (II. and III.) Oligonucleotides used in the individual steps are indicated and sequence representations in different shadings show the portions of the *PcLYS2* locus whose DNA sequence were determined in the individual steps. Restriction sites relevant for the experimental procedures are also indicated.

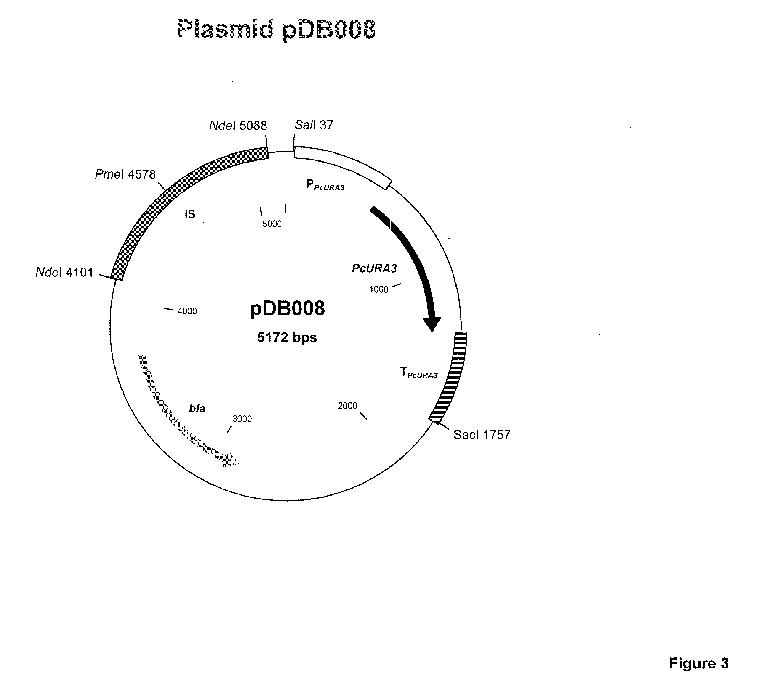

**Fig. 3** shows a graphical representation of the plasmid pDB008 for complementation of uracil-auxotrophic *Pichia ciferrii* mutants with the *Pichia ciferrii URA3* gene. The *PcURA3* promoter (white box), the *PcURA3* coding sequence (black arrow), the *PcURA3* terminator (horizontally hatched box), the ampicillin resistance gene (*bla;* light grey arrow) and the 5S-26S rDNA intergenic spacer with internal *Pme*I recognition site (IS; gridded box) are shown. Restriction sites relevant for the cloning procedures are also indicated.

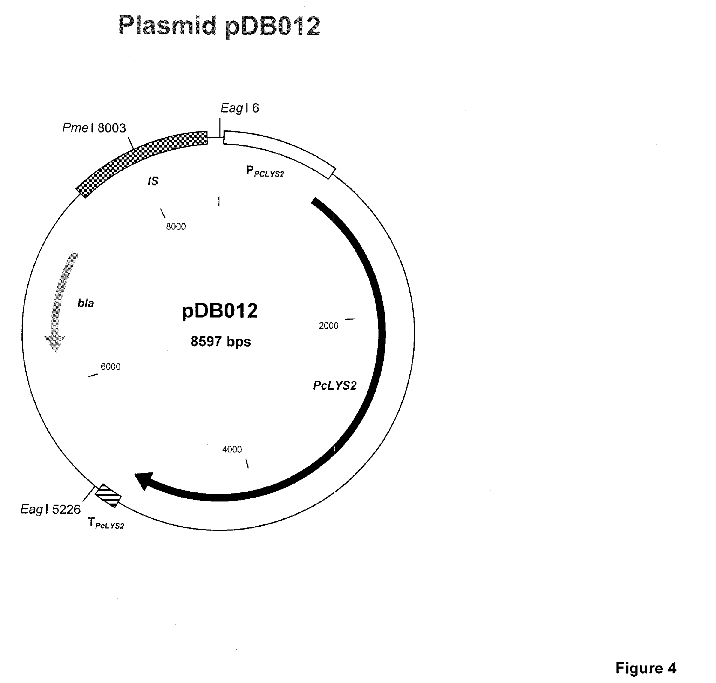

**Fig. 4** shows a graphical representation of the plasmid pDB012 for complementation of lysine-auxotrophic *Pichia ciferrii* mutants with the *Pichia ciferrii LYS2* gene. The *Pc LYS2* promoter (white box), the *Pc LYS2* coding sequence (black arrow), the *Pc LYS2* terminator (horizontally hatched box), the ampicillin resistance gene (*bla;* light grey arrow) and the 5S-26S rDNA intergenic spacer with internal *Pme*I recognition site (IS; gridded box) are shown. Restriction sites relevant for the cloning procedures are also indicated.

### EXAMPLES

### Example 1

### Isolation of genomic DNA from Pichia ciferrii F-60-10A NRRL 1031

*Pichia ciferrii* F-60-10A NRRL 1031 was grown in 50 ml YEPD medium (peptone 2% (w/v), yeast extract 1% (w/v) and glucose 2% (w/v) in 250 ml Erlenmeyer flasks at 200 rpm and 30°C and harvested after 18 h to an OD₆₀₀ of 1.5. Chromosomal DNA was isolated using the PUREGENE^{®} DNA Purification Kit for Yeast and Gram-positive bacteria (Gentra Systems Inc., cat.# D-6000A) according to the instructions of the manufacturer. A quality check of the isolated DNA by agarose gel electrophoresis demonstrated its high molecular weight (> 16 kbp).

### Example 2

### Isolation of uracil-auxotrophic mutants of Pichia ciferrii F-60-10A NRRL 1031

Mutations at the *URA3* locus of *Saccharomyces cerevisiae* can be obtained by a positive selection. Wild-type strains of yeast are unable to grow on medium containing the pyrimidine analog 5-fluoroorotic acid (5-FOA), whereas *ura3* mutants grow normally (Boeke et al. A positive selection for mutants lacking orotidine-5'-phosphate decarboxylase activity in yeast: 5-fluoro-orotic acid resistance. Molecular & General Genetics, 197: 345-346, 1984 and Boeke et al. 5-Fluoroorotic acid as a selective agent in yeast molecular genetics. Methods in Enzymology, 154: 164-175, 1987). Orotidine-5'-phosphate decarboxylase, encoded by *URA3,* converses 5-FOA to fluorodeoxyuridin, which serves as a toxic agent in yeast. In presence of uracil *ura3* mutants are able to grow on medium containing 5-FOA.

To select *ura3* mutants *Pichia ciferrii* F-60-10A NRRL 1031 was grown in 50 ml YEPD medium (peptone 2.0% (w/v), yeast extract 1.0% (w/v) and glucose 2.0% (w/v)) in 250 ml Erlenmeyer flasks at 250 rpm and 30°C to an OD₆₀₀ of 10.0, which corresponds to a titer of 1 x 10⁸ cells/ml. 5 x 10⁷ cells were plated on solid 5-FOA medium (glucose 4.0% (w/v), agar-agar 2.0% (w/v), yeast nitrogen base without amino acids 0.67% (w/v), uracil 2.0‰ (w/v) and 5-FOA 1.0% (w/v)) and incubated for 6 days at 30°C. About 50 colonies per plate appeared after the described incubation. They were analyzed with respect to uracil auxotrophy by transferring them to solid YNB medium without uracil (glucose 2.0% (w/v), agar-agar 2.0% (w/v), yeast nitrogen base without amino acids 0.67% (w/v)). Clones showing no growth on solid YNB medium without uracil were inoculated in 5 ml YEPD medium in test tubes to test for reversion of uracil auxotrophy in non-selective medium. After 3 days of growth at 250 rpm and 30°C the cultures reached stationary phase. 100 µl (3 x 10⁷ cells) of these cultures were plated on YNB medium lacking uracil. Those clones showing no growth (no reversion to uracil prototrophy) on solid YNB medium lacking uracil were referred to as uracil auxotrophic *Pichia ciferrii* strains lacking orotidine-5'-phosphate decarboxylase activity.

### Example 3

### Cloning and determination of the nucleotide sequence of the Pichia ciferrii URA3 gene

### Amplification of an internal part of the Pichia ciferrii URA3 gene

First, the amino acid sequences of putative orotidine-5'-phosphate decarboxylases from *Saccharomycotina* species were extracted from NCBI's database of completed and unfinished eukaryotic genomes (www.ncbi.nlm.nih.gov/sutils/genom_table.cgi) by performing a BLASTP search with the *Saccharomyces cerevisiae* Ura3p (GenBank acc.# NP_010893) as template. This protein has been characterized biochemically and been proven to represent an orotidine-5'-phosphate decarboxylase (Bell and Jones. Purification and characterization of yeast orotidine 5'-monophosphate decarboxylase overexpressed from plasmid PGU2. The Journal of Biological Chemistry, 266:12662-12667, 1991). The extracted sequences (all entries with E-values < 6 x 10⁻⁷¹) were aligned using the ClustalW program (www.ebi.ac.uk/clustalw). Suitable oligonucleotides for amplification of an internal part of the *Pichia ciferrii URA3* gene were derived by back-translating highly conserved stretches of amino acids within the Ura3p sequence taking into account the highly biased *Pichia ciferrii* codon usage. The following oligonucleotides were then synthesized by MWG Biotech (Ebersberg, Germany):
PcURA3-fw1:
   TTY GAA GAT MGW AAA TTY GCW (nt 1339-1359 in SEQ ID NO: 1)
   and
PcURA3-rv1:
   AAY AAA CCW CKA CCR ACR ATR AT (nt 1787-1765 in SEQ ID NO: 1).

These oligonucleotides were used to set up a PCR reaction according to Innis et al., (PCR protocols. A guide to methods and applications, 1990, Academic Press) with Herculase^{®} Hot Start PCR Master Mix (Stratagene, cat.# 600612) according to the instructions of the manufacturer and chromosomal DNA from *Pichia ciferrii* F-60-10A NRRL 1031 as template. A 449 bp fragment could be obtained by applying this method. The fragment was purified using the MinElute Gel Extraktion Kit (Qiagen, cat.# 28606) according to the instructions of the manufacturer.

### Determination of the DNA sequence of an internal part of the Pichia ciferrii URA3 gene

The DNA sequence of the purified PCR product was determined using the dideoxy chain termination method developed by Sanger et al. (Proceedings of the National Academy of Sciences, U.S.A., 74:5463-5467). As sequencing primer PcURA3-fw1 was used. DNA sequencing was performed by Sequiserve (Vaterstetten, Germany). The generated sequence information (449 bp, corresponding to nt 1339-1787 in SEQ ID NO: 1; Fig. 1) was translated into protein using the Clone Manager 7 software (Scientific & Educational Software) and the resulting amino acid sequence used as template for a BLASTP search with NCBI's non-redundant protein database (www.ncbi.nlm.nih.gov/BLAST/). The search resulted in the identification of *Kluyveromyces lactis* Ura3p (NCBI acc.# XP _454981) as being the protein in the database most similar to the new sequence, confirming that in fact portions of the *Pichia ciferrii URA3* ortholog had been amplified.

### Amplification of the entire Pichia ciferrii URA3 gene and determination of its DNA sequence

In order to determine the DNA sequence of the entire *Pichia ciferrii URA3* gene (coding sequence, promoter region and 3'-untranslated region) an inverse PCR approach was followed. Chromosomal DNA (300 ng) from *Pichia ciferrii* F-60-10A NRRL 1031 (isolated as described in Example 1) was digested overnight with *Hin*dIII (New England Biolabs, cat.# R0104L) according to the instructions of the manufacturer in a total volume of 50 µl. The digested DNA was purified using the QlAquick PCR Purification Kit (Qiagen, cat.# 28106) according to the instructions of the manufacturer. The eluted DNA (50 µl) was subjected to overnight ligation using the T4 DNA Ligase (New England Biolabs, cat.# M0202L) according to the instructions of the manufacturer in a total volume of 200 µl with 800 U of T4 DNA Ligase. The ligated DNA was purified using the QlAquick PCR Purification Kit according to the instructions of the manufacturer. 2.5 µl of the eluate was used as template for an inverse PCR reaction according to Innis et al., (PCR protocols. A guide to methods and applications, 1990, Academic Press). To that end two oligonucleotides hybridizing with the already known portion of the *Pichia ciferrii URA3* gene were used:
PcURA3-up-fw1:
   AAT ATG CAG GTG GTG CTT TCA AAA TT (nt 1385-1410 in SEQ ID NO: 1)
   and
PcURA3-up-rv1:
   GTG CTT TGA CTG TGT TCC CTA T (nt 1384-1363 in SEQ ID NO: 1).

Amplification was performed with Phusion^{™} High Fidelity PCR Master Mix according to the instructions of the manufacturer. Using this procedure a 0.6 kbp PCR product could be obtained. The fragment was purified using the MinElute PCR Purification Kit (Qiagen, cat.# 28006) according to the instructions of the manufacturer. The DNA sequence of this fragment was determined as described previously, using oligonucleotide PcURA3-up-fw1 as sequencing primer. The newly obtained sequence information covered nt 857-1338 in SEQ ID NO: 1. In order to obtain further information about the upstream region of the *Pichia ciferrii URA3* another round of inverse PCR had to be performed. Therefore, the above described experimental protocol was repeated, except that *Ssp*I (New England Biolabs, cat.# R0132S) was used for digesting *Pichia ciferrii* chromosomal DNA and the following oligonucleotides, synthesized by MWG Biotech (Ebersberg, Germany), were employed during inverse PCR:
PcURA3-up-fw2:
   CTT GTA CGG GAC ACG CTT CTA TG (nt 953-975 in SEQ ID NO: 1) and
PcURA3-up-rv2:
   TCT ATG GGA CGC CTA GTG TCT C (nt 888-867 in SEQ ID NO: 1).

Using this procedure a 1.0 kbp PCR product could be obtained. The fragment was purified using the MinElute PCR Purification Kit according to the instructions of the manufacturer. The DNA sequence of this fragment was determined as described previously with the oligonucleotide PcURA3-up-rv2 as sequencing primer. 856 bp of new sequence information (nt 1-856 in SEQ ID NO: 1) could be obtained which stretches to the next *Ssp*I site (Fig. 1). No new sequence information downstream of the already known DNA sequence could be obtained as the primers used for inverse PCR anneal upstream of the *Ssp*I site (Fig. 1). In order to obtain the DNA sequence of the 3'-end of the coding region of the *Pichia ciferrii URA3* gene and its 3'-untranslated region another round of inverse PCR had to be performed. Therefore, the above described experimental protocol was repeated, except that *Hin*dlll (New England Biolabs, cat.# R0104L) was used for digesting *Pichia ciferrii* chromosomal DNA and the following oligonucleotides, synthesized by MWG Biotech (Ebersberg, Germany), were employed during inverse PCR:
PcURA3-down-fw1:
   GTT GTT TCA ACT GGT AGT GAT ATC AT (nt 1741-1766 in SEQ ID NO: 1)
   and
PcURA3-down-rv1:
   GGT ATC AAC GGA TCT ATA TTG TTG A (nt 1740-1716 in SEQ ID NO: 1).

Using this procedure a 0.9 kbp PCR product could be obtained. The fragment was purified using the MinElute PCR Purification Kit according to the instructions of the manufacturer. The DNA sequence of this fragment was determined as described previously with the oligonucleotide PcURA3-down-fw1 as sequencing primer. 572 bp of new sequence information (nt 1788-2359 in SEQ ID NO: 1) could be obtained which stretches to the next *Hin*dIII site (Fig. 1). Using the described three-step procedure, a total of 2359 bp of the *Pichia ciferrii URA3* locus could be isolated and its DNA sequence be determined (see SEQ ID NO: 1 and Fig. 1).

The *Pichia ciferrii URA3* locus as depicted in Fig. 1 encodes the PcUra3p protein of 267 amino acids in length (SEQ ID NO: 2). PcUra3p has 76% (91%) and 78% (87%) positional amino acid identity (similarity) to hypothetical orotidine-5'-phosphate decarboxylases from *Debaromyces hansenii* (GenBank acc.# XP_456850) and *Candida glabrata* (GenBank acc.# XP_447384), respectively. Additionally the PcUra3p protein shows 75% identity and 89% similarity to *Candida albicans* orotidine-5'-phosphate decarboxylase, which has been shown to complement *Saccharomyces cerevisiae ura3* mutations (Losberger and Ernst. Sequence and transcript analysis of the C. albicans URA3 gene encoding orotidine-5'-phosphate decarboxylase. Current Genetics, 16: 153-158, 1989).

### Example 4

### Construction of a vector for complementation of uracil-auxotrophic Pichia ciferrii strains

In order to complement the uracil auxotrophy of the uracil auxotrophic *Pichia ciferrii* strain (see Example 2) we constructed an integrative *URA3* expression vector.

To that end 200 ng of chromosomal DNA of *Pichia ciferrii* F-60-10A NRRL 1031 (isolated as described in Example 1) was used as template for a PCR according to Innis et al. (PCR protocols. A guide to methods and applications, 1990, Academic Press) to amplify the promotor region of glyceraldehyde-3-phospate dehydrogenase of *Pichia ciferrii (TDH1).* For this the following oligonucleotides were applied:
pGAP-BgIII-for:
   5'-TAT ATA AGA TCT GTG GTA CCT ACA TAC AAT TGA CCC-3' (including a *Bgl*II-recognition sequence at the 5' end) and
pGAP-Ncol-rev:
   5'-TAT ATA CCA TGG TTA ATT AAT TAT TTG TTT GTT TG-3' (including a *Nco*I-recognition sequence at the 5' end).

The fragment was purified using the QlAquick PCR Purification Kit according to the instructions of the manufacturer. Then digestion of the PCR product with *Bgl*II and *Nco*I according to the instructions of the manufacturer of the restriction endonuclease: New England Biolabs, Schwalbach, Germany) yielded a 575 bp fragment, which was ligated into respectively cut pAG25 (Goldstein et al., Three new dominant gene disruption cassettes for gene disruption in Saccharomyces cerevisiae. Yeast, 15:1541-1553, 1999) creating vector pTH-GAP-nat1 (3892 bp) with the promotor region of glyceraldehyde-3-phospate dehydrogenase gene *(TDH3)* of *Pichia ciferrii* fused to *nat1.* The orientation and authenticity of the insert was determined by DNA sequencing. Ligation, preparation and transformation of chemically competent *Escherichia coli* cells was performed by methods known to the skilled person.

As the 5S-26S rDNA intergenic spacer (IS) of *Pichia ciferrii* was to be used as integration site for the plasmid, portions of the 5S-26S rDNA intergenic spacer (IS) were amplified by PCR using 200 ng of chromosomal DNA of *Pichia ciferrii* F-60-10A NRRL 1031 as template and following oligonucleotides:
pIS-Ndel-for:
   5'-TAT ATA CAT ATG CTA ATC ACA ACA GAA CAT TCT CTA ACG-3' (including a *Nde*I-recognition sequence at the 5' end) and
pIS-Ndel-rev:
   5'-TAT ATA CAT ATG GCT AGA TTG ACA GAA GTC GAT CAG-3' (including a *Nde*I-recognition sequence at the 5' end).

The fragment was purified using the QlAquick PCR Purification Kit according to the instructions of the manufacturer. Vector pTH-GAP-nat1 and the PCR product were digested with *Nde*I (according to the instructions of the manufacturer of the restriction endonuclease: New England Biolabs, Schwalbach, Germany) followed by ligation, creating vector pTH-GAP-nat1-IS2 (4864 bp). The orientation and authenticity of the insert was determined by DNA sequencing. Ligation, preparation and transformation of chemically competent *Escherichia coli* cells was performed by methods known to the skilled person.

To insert a single *Pme*I-recognition sequence for linearization of vector pTH-GAP-nat1-IS2, two fragments of the 5S-26S rDNA intergenic spacer (IS) of the *Pichia ciferrii* ribosomal RNA operon integrated into pTH-GAP-nat1-IS2 were amplified by PCR using vector pTH-GAP-natl-lS2 as template. Fragment 1 was amplified using oligonucleotides:
pIS-Ndel-rev:
   5'-TAT ATA CAT ATG GCT AGA TTG ACA GAA GTC GAT CAG-3' (including a *Nde*I-recognition sequence at the 5' end) and
PmeI-rv:
   5'-CCC ATC CAC TAA GTT TAA ACA CCC ATA CAA AAT CGA GCT TCA AAT C-3' (including a 21 bp sequence complementary to the oligonucleotide PmeI-fw and a *Pme*I-recognition sequence at the 5' end).
Fragment 2 was amplified using oligonucleotides:
p-IS-Ndel-for:
   5'-TAT ATA CAT ATG CTA ATC ACA ACA GAA CAT TCT CTA ACG-3' (including a *Nde*I-recognition sequence at the 5' end) and
PmeI-fw:
   5'-TGT TTA AAC TTA GTG GAT GGG AAA CCC TGT AGA ACT GGA CAA AC 3' (including a *Pme*I-recognition sequence at the 5' end).

The fragments were purified using the QlAquick PCR Purification Kit according to the instructions of the manufacturer. Finally, a fusion of fragments 1 and 2 was obtained by setting up a PCR with 10 ng of each of the two primary PCR products as templates with oligonucleotides:
p-IS-Ndel-for:
   5'-TAT ATA CAT ATG CTA ATC ACA ACA GAA CAT TCT CTA ACG-3' (including a *Nde*I-recognition sequence at the 5' end) and
plS-Ndel-rev:
   5'-TAT ATA CAT ATG GCT AGA TTG ACA GAA GTC GAT CAG-3' (including a *Nde*I-recognition sequence at the 5' end)
generating a 978 bp fragment with *Nde*I-recognition sequences at both ends and a *Pme*I-recognition sequence in the middle of the fragment.

The fragment was purified using the QlAquick PCR Purification Kit according to the instructions of the manufacturer. PCR product and vector pTH-GAP-nat1-IS2 were cut with *Nde*I (according to the instructions of the manufacturer of the restriction endonuclease: New England Biolabs, Schwalbach, Germany). Ligation was performed to generate vector pTH-GAP-nat1-IS2-Pmel (4879 bp). The orientation and authenticity of the insert was determined by DNA sequencing. Ligation, preparation and transformation of chemically competent *Escherichia coli* cells was performed by methods known to the skilled person.

To introduce the *Pichia ciferri URA3* gene including the native promoter and terminator the *URA3* gene was amplified using 200 ng of chromosomal DNA of *Pichia ciferrii* F-60-10A NRRL 1031 as template for a PCR with the following oligonucleotides:
PcURA3-Sall-fw:
   5'-TAT GTC GAC TTT GAT TGA TTT GAT ATA GAT TTG-3' (including a *Sal*I-recognition sequence at the 5' end) and
PcURA3-rv:
   5'-TGC TGG AGG GGT TCT TAA GTA TAT AG-3'.

The resulting 1752 bp fragment was purified using the MinElute PCR Purification Kit according to the instructions of the manufacturer. Then the PCR product was subjected to digestion with the restriction endonucleases *Sal*I and *Sac*I (according to the instructions of the manufacturer of the restriction endonucleases: New England Biolabs, Schwalbach, Germany). The digested 1703 bp fragment was gel-purified using the MinElute Gel Extraktion Kit according to the instructions of the manufacturer and ligated into the vector backbone of pTH-GAP-nat1-IS2-Pmel, which had been digested with *Sal*I and *Sac*I and been gel-purified (3448 bp), to generate vector pDB008 (5172 bp). The orientation and authenticity of the insert was determined by DNA sequencing. Ligation, preparation and transformation of chemically competent *Escherichia coli* cells was performed by methods known to the skilled person.

### Example 5

### Complementation of uracil-auxotrophic Pichia ciferrii strains with plasmid pDB008

To complement uracil-auxotrophic *Pichia ciferrii* cells, they were grown in YEPD medium to an OD₆₀₀ of 1 to 1.5. The cells were harvested by centrifugation and resuspended in 0.1 culture volume of 50 mM phosphate buffer (pH 7.5) to which 25 mM dithiothreitol was added. After incubation at 37°C for 15 min, the cells were washed twice with one culture volume of cold stabilization solution (270 mM sucrose, 10 mM Tris-HCI [pH 7.5], 1 mM MgCl₂), and resuspended in 0.01 culture volume of stabilization solution. 2.5 µl vector pDB008 linearized by digestion with *Pme*I (see Example 4) (containing 1.6 µg DNA) or, as a negative control, 2.5 µl H₂0 were mixed with 50 µl of cell suspension and incubated on ice for 10 min. Then the transformation mixture was transferred to a 2 mm electroporation cuvette. Electroporation was performed with a GenePulser Xcell at 500 V, 50 µF and 700 Ω (Bio-Rad Laboratories, Hercules, U.S.A.). After electroporation the cells were resuspended in 500 µl of stabilization solution and transferred to a culture tube containing 2 ml YPD medium. After overnight regeneration at 30°C and 250 rpm, aliquots of the regenerated cells were plated on YNB medium lacking uracil and incubated at 30°C. While no colonies appeared on plates containing the cells treated with water only, transformation of uracil-auxotrophic *Pichia ciferrii* cells with linearized vector pDB008 did result in formation of colonies on plates containing these cells after three days of incubation. Single colonies were analyzed for correct integration of plasmid pBD008 into the 5S-26S rDNA intergenic spacer via colony PCR. To that end the following oligonucleotides were synthesized by MWG Biotech (Ebersberg, Germany):
PcL41-up:
   GCG TTC TAA CGA CAA TAT GTC C
   and
26S rDNA-down:
   GTT TGT GTT TAT CAC AAC ACT TCT AAG.

These oligonucleotides were used to set up a PCR reaction according to Innis et al., (PCR protocols. A guide to methods and applications, 1990, Academic Press) with *Taq* PCR Master Mix (Qiagen, cat.# 201445) according to the instructions of the manufacturer and cells from uracil-prototrophic colonies as template. Only in case of correct integration of pDB008 in the 5S-26S rDNA intergenic spacer, the PCR reaction would yield a 1040 bp fragment, demonstrating that integration of vector pDB008 harbouring *Pichia ciferrii URA3* gene did take place at the 5S-26S rDNA intergenic spacer of *Pichia ciferrii.* All colonies tested led to amplification of the 1040 bp fragment. Consequently, transformation of uracil-auxotrophic *Pichia ciferrii* cells with vector pDB008 containing the *Pichia ciferrii URA3* gene resulted in complementation of the biosynthetic defect.

### Example 6

### Isolation of lysine auxotrophic mutants of Pichia ciferrii F-60-10A NRRL 1031

In contrast to wild-type strains of the yeast *Saccharomyces cerevisiae,* certain *lys* mutants are able to utilize *α*-aminoadipate as a primary source of nitrogen (Chattoo et al. α-Aminoadipate as a primary nitrogen source for Saccharomyces cerevisiae mutants. Genetics, 93: 51-65, 1979). The anabolism of high levels of *α*-aminoadipate through the biosynthetic pathway of lysine results in the accumulation of *α*-aminoadipic-δ-semialdehyde which serves as a toxic intermediate. The accumulation of this toxic intermediate is prevented by *lys2* and *lys5* mutations (Zaret and Sherman, Journal of Bacteriology, 162: 579-583, 1985) which both result in an inactive *α*-aminoadipate reductase enzyme.

To select *lys* mutants *Pichia ciferrii* F-60-10A NRRL 1031 was grown in 50 ml YEPD medium (peptone 2.0% (w/v), yeast extract 1.0% (w/v) and glucose 2.0% (w/v)) in 250 ml Erlenmeyer flasks at 250 rpm and 30°C to an OD₆₀₀ of 1.1, which corresponds to a titer of 1.1 x 10⁷ cells/ml. 2.2 x 10⁷ cells were plated on solid α-aminoadipate medium (glucose 2.0% (w/v), agar-agar 2.0% (w/v), yeast nitrogen base without amino acids and without nitrogen source 0.167% (w/v), L-lysine 3.0%o (w/v) and D,L-α-aminoadipate 0.15% (w/v)) and incubated for 6 days at 30°C. Several hundred *α*-aminoadipate-resistant colonies appeared after the described incubation. They were analyzed with respect to lysine auxotrophy by transferring them to solid YNB medium without lysine (glucose 2.0% (w/v), agar-agar 2.0% (w/v), yeast nitrogen base without amino acids 0.67% (w/v)). Clones showing no growth on solid YNB medium without lysine were inoculated in 5 ml YEPD medium in test tubes to test for phenotypic reversion of lysine auxotrophy in non-selective medium. After 4 days of growth at 250 rpm and 30°C cultures reached stationary phase. 100 µl of stationary cultures were used to inoculate new test tubes with 5 ml YEPD medium. After incubation of these cultures for 2 days at 250 rpm and 30°C, 200 µl were plated on solid YNB medium lacking lysine. Those clones showing no growth (no reversion to lysine prototrophy) on solid YNB medium lacking lysine were referred to as lysine auxotrophic *Pichia ciferrii* strains lacking α-aminoadipate reductase activity. The lack in these *Pichia ciferrii* strains of α-aminoadipate reductase activity may be caused by a loss-of-function mutation in the gene encoding α-aminoadipate reductase, *LYS2,* or by a loss-of-function mutation in the gene encoding α-aminoadipate reductase phosphopantetheinyl transferase, *LYS5.* The latter enzyme is required for post-translational modification of α-aminoadipate reductase, which is essential for α-aminoadipate reductase activity.

### Example 7

### Cloning and determination of the nucleotide sequence of the Pichia ciferrii LYS2 gene

### Amplification of an internal part of the Pichia ciferrii LYS2 gene

First, the amino acid sequences of putative α-aminoadipate reductases from *Saccharomycotina* species were extracted from NCBl's database of completed and unfinished eukaryotic genomes (www.ncbi.nlm.nih.gov/sutils/genom_table.cgi) by performing a BLASTP search with the *Saccharomyces cerevisiae* Lys2p (GenBank acc.# NP_009673) as template. The corresponding gene has been identified to represent the structural gene for α-aminoadipate reductase ( Sinha and Bhattacharjee. Lysine biosynthesis in Saccharomyces. Conversion of α-aminoadipate into α-aminoadipic-δ-semialdehyde. The Biochemical Journal, 125: 743-749, 1971), which catalyzes an essential step in the pathway for lysine biosynthesis in *Saccharomyces cerevisiae* and other fungi. The extracted sequences (all entries with E-values < 6 x 10⁻⁷³) were aligned using the ClustalW program (www.ebi.ac.uk/clustalw). Suitable oligonucleotides for amplification of an internal part of the *Pichia ciferrii LYS2* gene were derived by back-translating highly conserved stretches of amino acids within the Lys2p sequence taking into account the highly biased *Pichia ciferrii* codon usage. The following oligonucleotides were then synthesized by MWG Biotech (Ebersberg, Germany):
PcLYS2-fw:
   GTY ATY GAT CCW GCW TAY CCW CC (nt 1794-1816 in SEQ ID NO: 3) and
PcLYS2-rv:
   CCC AYT TWS WTT GAC CRT AAC C (nt 4293-4272 in SEQ ID NO: 3).

These oligonucleotides were used to set up a PCR reaction according to Innis et al., (PCR protocols. A guide to methods and applications, 1990, Academic Press) with Phusion^{™} High Fidelity PCR Master Mix (Finnzymes, cat.# F-531L) according to the instructions of the manufacturer and chromosomal DNA from *Pichia ciferrii* F-60-10A NRRL 1031 as template. A 2500 bp fragment could be obtained by applying this method. The fragment was purified using the MinElute Gel Extraktion Kit (Qiagen, cat.# 28606) according to the instructions of the manufacturer.

### Determination of the DNA sequence of an internal part of the Pichia ciferrii LYS2 gene

The DNA sequence of the purified PCR product was determined using the dideoxy chain termination method developed by Sanger et al. (Proceedings of the National Academy of Sciences, U.S.A., 74:5463-5467). As sequencing primers PcLYS2-fw, PcLYS2-rv and
PcLYS2-intrv:
   CTG AAT TGA GAA TCA TCT TGG (nt 3361-3341 in SEQ ID NO: 3)
were used. DNA sequencing was performed by Sequiserve (Vaterstetten, Germany). The generated sequence information (2500 bp, corresponding to nt 1794-4293 in SEQ ID NO: 3; Fig. 2) was translated into protein using the Clone Manager 7 software (Scientific & Educational Software) and the resulting amino acid sequence used as template for a BLASTP search with NCBI's non-redundant protein database (www.ncbi.nlm.nih.gov/BLAST/). The search resulted in the identification of *Candida albicans* Lys2p (NCBI acc.# EAL02670), as being the protein in the database most similar to the new sequence, confirming that in fact portions of the *Pichia ciferrii LYS2* ortholog had been amplified.

### Amplification of the entire Pichia ciferrii LYS2 gene and determination of its DNA sequence

In order to determine the DNA sequence of the entire *Pichia ciferrii LYS2* gene (coding sequence, promoter region and 3'-untranslated region) an inverse PCR approach was followed. Chromosomal DNA (300 ng) from *Pichia ciferrii* F-60-10A NRRL 1031 (isolated as described in Example 1) was digested overnight with *Eco*32I (MBI Fermentas, cat.# ER0301) according to the instructions of the manufacturer in a total volume of 50 µl. The digested DNA was purified using the QlAquick PCR Purification Kit (Qiagen, cat.# 28106) according to the instructions of the manufacturer. The eluted DNA (50 µl) was subjected to overnight ligation using the T4 DNA Ligase (New England Biolabs, cat.# M0202L) according to the instructions of the manufacturer in a total volume of 200 µl with 800 U of T4 DNA Ligase. The ligated DNA was purified using the QlAquick PCR Purification Kit according to the instructions of the manufacturer. 2.5 µl of the eluate was used as template for an inverse PCR reaction according to Innis et al., (PCR protocols. A guide to methods and applications, 1990, Academic Press). To that end two oligonucleotides hybridizing with the already known portion of the *Pichia ciferrii LYS2* gene were applied:
PcLYS2-us-fw:
   CAT CAA AGA TGA ATT AGA TGT TGT TTC (nt 1910-1936 in SEQ ID NO: 3) and
PcLYS2-us-rv:
   CAA CAA GTT CAT CCA AGA CAC C (nt 1902-1881 in SEQ ID NO: 3).

Amplification was performed with Phusion^{™} High Fidelity PCR Master Mix according to the instructions of the manufacturer. Using this procedure a 4.0 kbp PCR product could be obtained. The fragment was purified using the MinElute PCR Purification Kit (Qiagen, cat.# 28006) according to the instructions of the manufacturer. The DNA sequence of this fragment was determined as described previously, using oligonucleotides PcLYS2-us-rv and
PcLYS2-us-rv2:
   CCT TCA ACT TTC GAT ATT ATA G (nt 976-955 in SEQ ID NO: 3)
as sequencing primer. The newly obtained sequence information covered nt 1-1793 in SEQ ID NO: 3. No new sequence information downstream of the already known DNA sequence could be obtained as the primers used for inverse PCR anneal upstream of the *E*co32I site (Fig. 2). In order to obtain the DNA sequence of the 3'-end of the coding region of the *Pichia ciferrii LYS2* gene and its 3'-untranslated region another round of inverse PCR had to be performed. Therefore, the above described experimental protocol was repeated, except that *Mun*I (MBI Fermentas, cat.# ER0751) was used for digesting *Pichia ciferrii* chromosomal DNA and the following oligonucleotides, synthesized by MWG Biotech (Ebersberg, Germany), were employed during inverse PCR:
PcLYS2-ds-fw:
   TAA AGA TGG TAT TCC AGA ATC TGA TG (nt 4214-4239 in SEQ ID NO: 3) and
PcLYS2-ds-rv:
   AGA TAA TTC AAC GAA ATG TTG AGT ATC (nt 4193-4167 in SEQ ID NO: 3).

Using this procedure a 1.9 kbp PCR product could be obtained. The fragment was purified using the MinElute PCR Purification Kit according to the instructions of the manufacturer. The DNA sequence of this fragment was determined as described previously with oligonucleotides PcLYS2-ds-fw and
PcLYS2-ds-fw2:
   ACC TTT GTA CAT ATG AAA TCT C (nt 5138-5159 in SEQ ID NO: 3)
as sequencing primers. 1608 bp of new sequence information (nt 4294-5900 in SEQ ID NO: 3) could be obtained which stretches to the next *Mun*I site (Fig. 2). Using the described three-step procedure, a total of 5900 bp of the *Pichia ciferrii LYS2* locus could be isolated and its DNA sequence be determined (see SEQ ID NO: 3 and Fig. 2).

The *Pichia ciferrii LYS2* locus as depicted in Fig. 2 encodes the PcLys2p protein of 1395 amino acids in length (SEQ ID NO: 4). PcLys2p has 65% (79%) and 61% (75%) positional amino acid identity (similarity) to the α-aminoadipate reductase from *Candida albicans* (GenBank acc.# EAL02670) and the hypothetical α-aminoadipate reductase from *Kluyveromyces lactis* (GenBank acc.# XP _451939), respectively. The *Candida albicans LYS2* gene has been shown to complement *Saccharomyces cerevisiae lys2* mutations (Suvarna et al. Molecular analysis of the LYS2 gene of Candida albicans: homology to peptide antibiotic synthetases and the regulation of the α-aminoadipate reductase. Current Genetics, 33: 268-275, 1998). It has been characterized biochemically and been shown to have α-aminoadipate reductase activity (Guo et al. Novel posttranslational activation of the LYS2-encoded α-aminoadipate reductase for biosynthesis of lysine and site-directed mutational analysis of conserved amino acid residues in the activation domain of Candida albicans. Journal of Bacteriology, 183: 7120-7125, 2001). Additionally the PcLys2p contains a highly conserved phosphopantetheinylation domain (LGGHSI, amino acid residues 881 to 886 in SEQ ID NO: 4) (Suvarna et al. Molecular analysis of the LYS2 gene of Candida albicans: homology to peptide antibiotic synthetases and the regulation of the α-aminoadipate reductase. Current Genetics, 33: 268-275, 1998).

### Example 8

### Construction of a vector for complementation of lysine-auxotrophic Pichia ciferrii strains

In order to complement the lysine auxotrophy of the lysine auxotrophic *Pichia ciferrii* strain (see example 6) we constructed an integrative *LYS2* expression vector.

To that end 500 ng of chromosomal DNA of *Pichia ciferrii* F-60-10A NRRL 1031 (isolated as described in Example 1) was used as template for a PCR according to Innis et al. (PCR protocols. A guide to methods and applications, 1990, Academic Press) to amplify the α-aminoadipate reductase gene (coding sequence including promoter and terminator) of *Pichia ciferrii (LYS2).* For this the following oligonucleotides were applied:
PcLYS2-compl-fw (nt 1-36 in SEQ ID NO: 3):
   5'-AAT TAT TCT TAT CTT TAA CAT CAT TAC CAC TTA ACC-3' and
PcLYS2-compl-rv (nt 5158-5117 in SEQ ID NO: 3):
   5'-AGA TTT CAT ATG TAC AAA GGT TAA TAC ACA TAT AAT ATA TTC-3'.

The fragment was purified using the QlAquick PCR Purification Kit according to the instructions of the manufacturer. Ligation into vector pCR^{®}-Blunt II-TOPO^{®} and transformation of chemically competent *Escherichia coli* cells was performed according to the Zero Blunt^{®} TOPO^{®} PCR Cloning Kit (Invitrogen Corporation, Carlsbad). The orientation and authenticity of the insert was determined by DNA sequencing.

The resulting vector was digested with Eagl (New England Biolabs, cat.# R0505S) according to the instructions of the manufacturer, followed by gel extraction of the 5220 bp insert carrying the *Pichia ciferrii LYS2* gene using QlAquick Gel Extraktion Kit (Qiagen, cat.# 28706) according to the instructions of the manufacturer and ligation of the 5220 bp insert into the backbone of vector pTH-GAP-nat1-IS2-Pmel (described in example 4) obtained by digestion with *Eag*I (3377 bp), generating vector pDB012. The orientation and authenticity of the insert was determined by restriction analysis. Ligation, preparation and transformation of chemically competent *Escherichia coli* cells was performed by methods known to the skilled person.

### Example 9

### Complementation of lysine-auxotrophic Pichia ciferrii strains with plasmid pDB012

To complement lysine-auxotrophic *Pichia ciferrii* cells, they were grown in YEPD medium to an OD₆₀₀ of 1 to 1.5. The cells were harvested by centrifugation and resuspended in 0.1 culture volume of 50 mM phosphate buffer (pH 7.5) to which 25 mM dithiothreitol was added. After incubation at 37°C for 15 min, the cells were washed twice with a culture volume of cold stabilization solution (270 mM sucrose, 10 mM Tris-HCI [pH 7.5], 1 mM MgCI₂), and resuspended in 0.01 culture volume of stabilization solution. 2.5 µl vector pDB012 linearized by digestion with *Pme*I (see Example 8) (containing 1.0 µg DNA) or, as a negative control, 2.5 µl H₂0 were mixed with 50 µl of cell suspension and incubated on ice for 10 min. Then the transformation mixture was transferred to a 2 mm electroporation cuvette. Electroporation was performed with a GenePulser Xcell at 500 V, 50 µF and 700 Ω (Bio-Rad Laboratories, Hercules, U.S.A.). After electroporation the cells were resuspended in 500 µl of stabilization solution and transferred to a culture tube containing 2 ml YPD medium. After overnight regeneration at 30°C and 250 rpm, aliquots of the regenerated cells were plated on YNB medium lacking lysine and incubated at 30°C. While no colonies appeared on plates containing the cells treated with water only, transformation of lysine-auxotrophic *Pichia ciferrii* cells with linearized vector pDB012 did result in formation of colonies on plates containing these cells after three days of incubation. Single colonies were analyzed for correct integration of plasmid pBD012 into the 5S-26S rDNA intergenic spacer via colony PCR. To that end the following oligonucleotides were synthesized by MWG Biotech (Ebersberg, Germany):
PcL41-up:
   GCG TTC TAA CGA CAA TAT GTC C
   and
26S rDNA-down:
   GTT TGT GTT TAT CAC AAC ACT TCT AAG.

These oligonucleotides were used to set up a PCR reaction according to Innis et al., (PCR protocols. A guide to methods and applications, 1990, Academic Press) with *Taq* PCR Master Mix (Qiagen, cat.# 201445) according to the instructions of the manufacturer and cells from lysine-prototrophic colonies as template. Only in case of correct integration of pDB012 in the 5S-26S rDNA intergenic spacer, the PCR reaction would yield a 1040 bp fragment, demonstrating that integration of vector pDB012 harbouring the *Pichia ciferrii LYS2* gene did take place at the 5S-26S rDNA intergenic spacer of *Pichia ciferrii.* All colonies tested led to amplification of the 1040 bp fragment. Consequently, transformation of lysine-auxotrophic *Pichia ciferrii* cells with vector pDB012 containing the *Pichia ciferrii LYS2* gene resulted in complementation of the biosynthetic defect.

## Claims

1. A *Pichia ciferrii* cell that is defective in a biosynthetic pathway.

2. The *Pichia ciferrii* cell of claim 1 wherein the biosynthetic pathway is a pathway for a nucleotide, an amino acid and/or a vitamin.

3. The *Pichia ciferrii* cell of claim 1 or 2, wherein the biosynthetic pathway is a pathway for uracil and/or for lysine.

4. The *Pichia ciferrii* cell of claim 3, wherein the biosynthetic pathway for uracil is defective in the activity of the enzyme orotidine-5-phosphate decarboxylase.

5. A *Pichia ciferrii* cell of claim 3, wherein the biosynthetic pathway for lysine is defective in the activity of the enzyme α-aminoadipate reductase.

6. A *Pichia ciferrii* transformation system comprising the *Pichia ciferrii* cell of any of the preceding claims as a host cell and a polynucleotide comprising a functional gene that complements the defect in the biosynthetic pathway in which the host cell is defective.

7. The *Pichia ciferrii* transformation system of claim 6 wherein the functional gene is the *URA3* gene and/or the *LYS2* gene, preferably from *Pichia ciferrii.*

8. A method for transformation of *Pichia ciferrii* comprising transforming the *Pichia ciferrii* cell of any one of the claims 1-5 with a polynucleotide comprising a functional gene that complements the defect in the biosynthetic pathway and isolating transformed cells that are prototrophic for the defect in the biosynthetic pathway .

9. The method according to claim 8 wherein the functional gene is the *URA3* gene and/or the *LYS2* gene, preferably from *Pichia ciferrii.*

10. A polynucleotide for use in a method according to claim 8 or 9 comprising a functional gene from *Pichia ciferrii* that complements the defect in said biosynthetic pathway in which the host cell is defective.

11. A polynucleotide for use in a method according to claim 8 or 9 comprising a functional gene that complements the defect in said biosynthetic pathway in which the host cell is defective, wherein the functional gene has a DNA sequence encoding a polypeptide with an amino acid sequence of SEQ ID NO: 2, an amino acid sequence of SEQ ID NO: 4, an amino acid sequence having a degree of identity of at least 91% to the amino acid sequence of SEQ ID NO: 2 or an amino acid sequence having a degree of identity of at least 70% to the amino acid sequence of SEQ ID NO: 4, preferably having a DNA sequence according to SEQ ID NO: 1 or SEQ ID NO: 2.

12. A method for the production of a compound of interest comprising transforming the *Pichia ciferrii* cell of any one of claims 1-5 with a polynucleotide containing a functional gene complementing the defect in said biosynthetic pathway in which the host cell is defective and a polynucleotide comprising a DNA sequence of interest, selecting transformed cells for prototrophy, culturing transformed cells under conditions conducive to production of the compound of interest, and, optionally, recovering the compound of interest.

13. The method according to claim 12 wherein the functional gene is the *URA3* gene and/or the *LYS2* gene, preferably from *Pichia ciferrii.*

14. A polypeptide that complements a defect in the biosynthetic pathway for uracil or lysine of *Pichia ciferrii* and has an amino acid sequence of SEQ ID NO: 2 or SEQ ID NO: 4, respectively, or an amino acid sequence having a degree of identity of at least 91 % to the amino acid sequence of SEQ ID NO: 2 or an amino acid sequence having a degree of identity of at least 70% to the amino acid sequence of SEQ ID NO: 4.
